# EUROPEAN PATENT APPLICATION

(11) **EP 4 659 712 A1**
(43) Date of publication of application: **10.12.2025**
(21) Application number: 25180687.3
(22) Date of filing: 04.06.2025
(51) Int. Cl.: A61F 2/44, A61F 2/30, A61B 17/80, A61F 2/42, A61F 2/28

(54) **EXPANDABLE COTTON AND EVANS IMPLANTS**

(30) Priority: 06.06.2024 US 202418735420
(71) Applicant: Globus Medical, Inc., Audubon, PA 19403 (US)
(72) Inventor: SCHEINFIELD, Richard, PHILADELPHIA, 19131 (US); NORTON, Garret, PHOENIXVILLE, 19460 (US); SANDOE, Jessica, LANSDALE, 19446 (US); RUSH, Jesse, SCHWENKSVILLE, 19473 (US)
(74) Representative: Morabito, Sara

(57) **Abstract**

Expandable wedge implants and related methods. The expandable implant may include a bottom endplate, a top endplate pivotably connected to the bottom endplate, an actuator with curved ramps configured to mate with angled ramps on the top endplate, and a drive screw configured to move the actuator to thereby expand the top endplate relative to the bottom endplate. The expandable implants may be configured to correct pes planus or a flatfoot deformity using Evans and/or Cotton procedures during foot surgery.

## Description

### FIELD OF THE INVENTION

The present disclosure generally relates to devices and methods for correcting a bone deformity, and more particularly relates to an expandable orthopedic implant capable of being inserted into an osteotomy incision to reposition and stabilize a portion of bone.

### BACKGROUND OF THE INVENTION

In skeletally mature adults, pes planus or flatfoot deformity may be characterized by a collapse of the medial longitudinal arch of the foot, heel valgus deformity, and prominence of the medial talar bone, thereby causing the entire sole of the foot to contact or nearly contact the ground when standing. The deformity may be corrected using an Evans procedure by creating an osteotomy or single cut of the calcaneus bone of the foot and inserting a static wedge or spacer to lengthen the lateral column of the foot. The process of inserting the wedge corrects the valgus heel deformity by medializing the portion of the calcaneus posterior to the osteotomy. A Cotton procedure, or medial cuneiform osteotomy, may also be used as an adjunctive flatfoot reconstructive procedure to correct forefoot varus deformity after rearfoot fusion or osteotomy. The Cotton procedure may involve creating an osteotomy or single cut at the midpoint of the dorsal aspect of the medial cuneiform bone parallel to the first tarsometatarsal joint, keeping the plantar cortex intact to create a hinge. A static wedge or spacer is then inserted to plantarflex the forefoot to recreate the natural medial longitudinal arch of the foot.

Static wedges require the use of trials to determine the optimal wedge footprint and thickness required to correct the flatfoot deformity. In order to choose the optimal wedge thickness, multiple trials may need to be inserted and removed from the osteotomy site. This can lead to deformation of the cortical walls and increase the risk of post-operative implant subsidence into the softer cancellous bone making up a majority of the calcaneus and medial cuneiform bones.

For a correction requiring the use of a thicker wedge, a distraction may be necessary. The process of insertion into the distracted osteotomy site can acutely damage or break off bone on the anterior or posterior calcaneal or medial cuneiform fragments surrounding the wedge. There is potential for this damage to affect the surgeon's correction plan by forcing the surgeon to use a different wedge size than previously planned, prolonging procedure time, and/or causing excessive distraction stress on the calcaneal and/or medial cuneiform bone fragments.

There are instances where post-operative bony resorption around the implant may lead to lost correction and the need for a revision procedure to insert a thicker wedge. Revision surgery increases the risk of wound healing complications at and around the incision area, for which the soft tissues covering the lateral calcaneus and dorsal medial cuneiform are especially susceptible.

As such, there exists a need for orthopedic implants capable of being installed in an osteotomy incision to correct a foot deformity, while also addressing the issues of trialing, damage to the bone fragments due to distraction, and/or revision complications.

### SUMMARY OF THE INVENTION

To meet this and other needs, implants and methods for repositioning and stabilizing a portion of bone are provided. In particular, expandable orthopedic implants, for example, for foot surgery may be used to treat pes planus or flatfoot and other patient indications. The expandable implants may be capable of continuous expansion within a thickness range, able to automatically lock at the desired thickness, and may be offered in a range of footprint options. The expandable technology allows a surgeon to dial in the wedge thickness in order to achieve the desired lengthening of the lateral column of the foot for an Evans procedure, and/or plantarflexion of the forefoot for a Cotton procedure, to correct the deformity.

According to one embodiment, an expandable implant includes a bottom endplate and a top endplate pivotably connected to the bottom endplate, the top endplate defining a pair of angled ramps, an actuator including a pair of wings with arced ramps configured to mate with the respective angled ramps on the top endplate, and a drive screw configured to move the actuator. Rotation of the drive screw translates the actuator to expand the top endplate away from the bottom endplate. The arced ramps on the actuator mate with the angled ramps on the top endplate to achieve tangential surface contact at any degree of implant expansion.

The expandable implant may include one or more of the following features. The actuator may include a prominent cylindrical portion extending from its center and defining a threaded through bore configured to receive the drive screw therethrough. The actuator may include a pair of tabs configured to fit within female channels defined in the angled ramps of the top endplate to retain the actuator to the top endplate. The top endplate may be pivotably connected to the bottom endplate with a hinge pin. When expanded, the top endplate may be angled relative to the bottom endplate. The bottom endplate may define a first bore configured to receive a portion of the drive screw and a second bore configured to receive a bone screw. The drive screw may include a threaded shaft, a distal tip with a reduced diameter configured to fit in a pocket in the bottom endplate, and an enlarged head with a drive recess.

According to one embodiment, an expandable implant includes a bottom endplate having a front end, an opposite rear end, and a pair of sidewalls connecting the front and rear ends and defining opposite sidewall grooves, a top endplate pivotably connected to the front end of the bottom endplate, the top endplate having an outer surface with a plurality of teeth configured to engage bone and an inner surface defining a pair of ramps, an actuator including a center slot and non-threaded bore, a pair of pivot slots on opposite sides of the center slot, and a pair of outwardly extending tabs receivable in the grooves in the bottom endplate to guide translation of the actuator, a pair of actuator pivots, each pivot having a ring receivable within the respective pivot slot of the actuator and secured via a pin and a foot configured to mate with one of the ramps on the top endplate, and a drive screw positioned through the non-threaded bore in the actuator and threadedly engaged with the bottom endplate. Rotation of the drive screw pulls the actuator forward, thereby causing the two actuator pivots to slide along the ramps of the top endplate to expand the top endplate away from the bottom endplate.

The expandable implant may include one or more of the following features. The foot of the actuator pivot may define a male projection with a sliding surface which mates with a corresponding sliding surface within a female portion of the ramp in the top endplate. The actuator pivots may be able to freely rotate on a cylindrical outer surface of the respective pin, which provides continuous contact between the sliding surfaces of the actuator pivots and the top endplate when angulation between the top endplate and bottom endplate changes. The pivot pins may have an interference fit with one side of the pivot slot and a clearance fit with the actuator pivot to allow the pivot to freely rotate about the pivot pin. The drive screw may include an enlarged head and a threaded shaft, and a proximal-most portion of the head may be recessed to form a collar, which is configured to seat within a corresponding pocket in a friction ring. The head of the drive screw may seat into the pocket in the friction ring and fit into the center slot of the actuator behind the non-threaded bore such that the friction ring is compressed on two sides by the center slot, which provides resistance when rotating the drive screw. The top endplate may be pivotably connected to the bottom endplate with a hinge pin, and the hinge pin may have an interference fit with one side of the bottom endplate for retention and a clearance fit with the top endplate to allow the top endplate to freely rotate about the hinge pin.

According to one embodiment, a method of correcting a bone deformity may include one or more of the following steps in any suitable order: (1) forming an osteotomy in bone; (2) inserting an expandable implant into the osteotomy, the expandable implant having a bottom endplate, a top endplate pivotably connected to the bottom endplate, an actuator with curved ramps configured to engage with angled ramps on the top endplate, and a drive screw configured to move the actuator; and (3) expanding the implant to correct the bone deformity by rotating the drive screw to translate the actuator, thereby expanding the top endplate away from the bottom endplate, wherein the curved ramps on the actuator mate with the angled ramps on the top endplate to achieve tangential surface contact at any degree of implant expansion. The method may further include: (4) inserting one or two bone screws through the top and/or bottom endplates to secure the implant to adjacent bone. The expandable implant may have an adjustable wedge thickness configured to be dialed in by a surgeon to correct the deformity. The bone deformity may be a flatfoot deformity. The osteotomy may be formed at a midpoint of a dorsal aspect of a medial cuneiform bone during a Cotton procedure. Alternatively, the osteotomy may be formed in a calcaneus bone during an Evans procedure.

According to yet another embodiment, a method of correcting a flatfoot deformity may include one or more of the following steps in any suitable order: (1) creating a first osteotomy on the lateral aspect of the calcaneus bone; (2) inserting a first expandable implant into the first osteotomy site; (3) expanding the first expandable implant, thereby medializing a portion of the calcaneus posterior to the first osteotomy; (4) creating a second osteotomy on a dorsal aspect of the medial cuneiform bone; (5) inserting a second expandable implant into the second osteotomy site; and (6) expanding the second expandable implant, thereby creating a plantarflexion of the forefoot to recreate a natural medial longitudinal arch of the foot. In both instances, the expandable implants do not require trialing or independent distraction prior to use, which minimizes damage and helps to preserve the bones.

According to yet another embodiment, a kit may include a plurality of expandable implants of different sizes and configurations. The kit may further include bone fasteners, such as locking speed screws, of different types and sizes, and one or more devices suitable for installing and/or removing the implants and systems described herein, such as insertion devices or drivers, and other tools and devices, which may be suitable for surgery.

### BRIEF DESCRIPTION OF THE DRAWINGS

The present embodiments will become more fully understood from the detailed description and the accompanying drawings, wherein:
FIGS. 1A-1C illustrate top and bottom perspective views of an expandable orthopedic implant configured for a Cotton procedure in an expanded position according to one embodiment;
FIGS. 2A-2C show top and rear views, respectively, of the expandable orthopedic implant of FIGS. 1A-1C indicating the depth, width, and thickness of the implant;
FIG. 3 shows an exploded view of the expandable orthopedic implant of FIGS. 1A-1C;
FIGS. 4A-4B show an actuator having arced ramps configured to mate with angled ramps on the top endplate according to one embodiment;
FIGS. 5A-5B show the bottom and top surfaces of the top endplate with angled ramps configured to expand the top endplate according to one embodiment;
FIGS. 6A-6C show a polyaxial hole defined through the bottom endplate configured for placement of a locking speed screw according to one embodiment;
FIGS. 7A-7C show side views of implant in collapsed, half-way expanded, and fully expanded positions, respectively;
FIGS. 8A-8C show cross-sectional views of the implant and tangential surface engagement of the actuator and top endplate in the collapsed, half-way expanded, and fully expanded positions, respectively;
FIGS. 9A-9C illustrate top and bottom perspective views of an expandable orthopedic implant configured for an Evans procedure in an expanded position according to one embodiment;
FIGS. 10A-10C show top and rear views, respectively, of the expandable orthopedic implant of FIGS. 9A-9C indicating the depth, height, and thickness of the implant;
FIG. 11 shows an exploded view of the expandable orthopedic implant of FIGS. 9A-9C;
FIGS. 12A-12B show an actuator having arced ramps configured to mate with the angled ramps on the top endplate according to one embodiment;
FIGS. 13A-13B show bottom and top surfaces of the top endplate with the angled ramps configured to engage with the actuator to expand the top endplate according to one embodiment;
FIGS. 14A-14C show upper and lower polyaxial holes in the top and bottom endplates, respectively, which are configured for placement of respective locking speed screws according to one embodiment;
FIGS. 15A-15B show the expandable implant in a collapsed configuration with the upper and lower locking speed screws configured to fixate the implant to posterior and anterior calcaneal bone fragments according to one embodiment;
FIGS. 16A-16C show side views of implant in collapsed, half-way expanded, and fully expanded positions, respectively;
FIGS. 17A-17C show cross-sectional views of the implant and tangential surface engagement of the actuator and top endplate in the collapsed, half-way expanded, and fully expanded positions, respectively;
FIG. 18 shows an example of a foot anatomy and the expandable orthopedic Evans implant of FIG. 9A-9C implanted into an osteotomy site in the calcaneus according to one embodiment;
FIGS. 19A-19C illustrate top and bottom perspective views of an expandable orthopedic implant configured for a Cotton procedure in an expanded position according to one embodiment;
FIGS. 20A-20C show top and rear views, respectively, of the expandable orthopedic implant of FIGS. 19A-19C indicating the depth, width, and thickness of the implant;
FIG. 21 shows an exploded view of the expandable orthopedic implant of FIGS. 19A-19C;
FIG. 22 shows an actuator having slots configured to receive actuator pivots, which expand the top endplate according to one embodiment;
FIGS. 23A-23B show actuator pivots having a ring and foot configured to expand the top endplate according to one embodiment;
FIGS. 24A-24B show bottom and top perspective views, respectively, of the top endplate with ramped slots configured to expand the top endplate according to one embodiment;
FIGS. 25A-25B show the drive screw and friction ring, respectively, according to one embodiment;
FIGS. 26A-26C show side views of implant in collapsed, half-way expanded, and fully expanded positions, respectively;
FIGS. 27A-27C show cross-sectional views of the implant and sliding surface engagement of the pivot actuator and top endplate in the collapsed, half-way expanded, and fully expanded positions, respectively;
FIGS. 28A-28C show cross-sectional views of the implant and movement of the drive screw, actuator, and top endplate in the collapsed, half-way expanded, and fully expanded positions, respectively; and
FIG. 29 shows the actuator bottomed out against the bottom endplate once fully expanded according to one embodiment.

### DETAILED DESCRIPTION

Embodiments of the disclosure are generally directed to devices and methods for repositioning and stabilizing a portion of bone. Specifically, expandable orthopedic implants are configured to expand in thickness to properly orient the bone, stabilize the bone, and/or provide a fusion site for bone to grow. The expandable implant may be inserted into an osteotomy site and expanded to distract and stabilize the osteotomy space. The osteotomy and expanded implant may help to reshape or realign bone to relieve pain and discomfort and/or correct a deformity. For example, the expandable implant may be configured to correct pes planus or a flatfoot deformity using an Evans procedure in the calcaneus during foot surgery. In addition, or alternatively, the expandable implant may be configured to correct a deformity using a Cotton procedure in the cuneiform during foot surgery.

The Evans procedure may include creating an osteotomy or single cut on the lateral aspect of the calcaneus bone and inserting and expanding the expandable implant to lengthen the lateral column of the foot. The process of inserting and expanding the implant fixes the valgus heel deformity by medializing the portion of the calcaneus posterior to the osteotomy. The expandable implant allows the surgeon to dial in the wedge thickness in order to achieve the desired lengthening of the lateral column of the foot to correct the deformity.

The Cotton procedure may include creating an osteotomy or single cut at the midpoint of the dorsal aspect of the medial cuneiform bone parallel to the first tarsometatarsal joint, keeping the plantar cortex intact to create a hinge. The process of inserting and expanding the implant causes plantarflexion of the forefoot to recreate the natural medial longitudinal arch of the foot. The expandable implant allows the surgeon to dial in the wedge thickness in order to achieve the desired plantarflex of the forefoot to correct the deformity.

Although generally described herein for use in Evans and/or Cotton foot procedures, it will be readily appreciated by those skilled in the art that the expandable implant may be employed in any number of suitable orthopedic approaches and procedures. The implant may be used for internal fixation of bone fractures, fusions, and/or osteotomies, including but not limited to, metatarsal/cuneiform arthrodesis, tibial osteotomies, femoral osteotomies, pelvic osteotomies, vertebral osteotomies or resections, as well as other indications to correct deformities, relieve pain, and/or restore function.

Components of all of the devices disclosed herein may be manufactured of any suitable materials including metals (e.g., titanium), metal alloys (e.g., stainless steel, cobalt-chromium, and titanium alloys), ceramics, plastics, plastic composites, or polymeric materials (e.g., polyether ether ketone (PEEK), polyphenylene sulfone (PPSU), polysulfone (PSU), polycarbonate (PC), polyetherimide (PEI), polypropylene (PP), polyacetals, or mixtures or co-polymers thereof), and/or combinations thereof. In some embodiments, the devices may include radiolucent and/or radiopaque materials. The components can also be machined and/or manufactured using any suitable techniques (e.g., 3D printing).

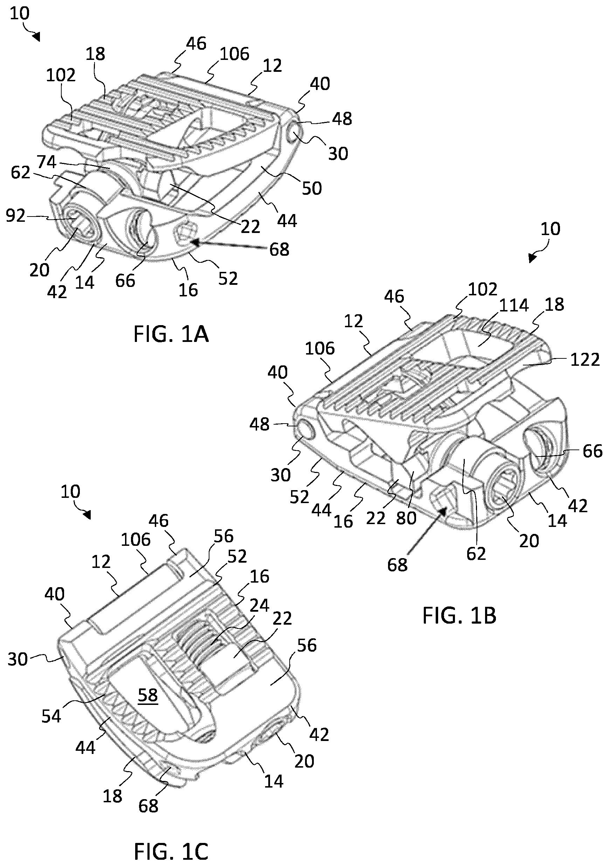

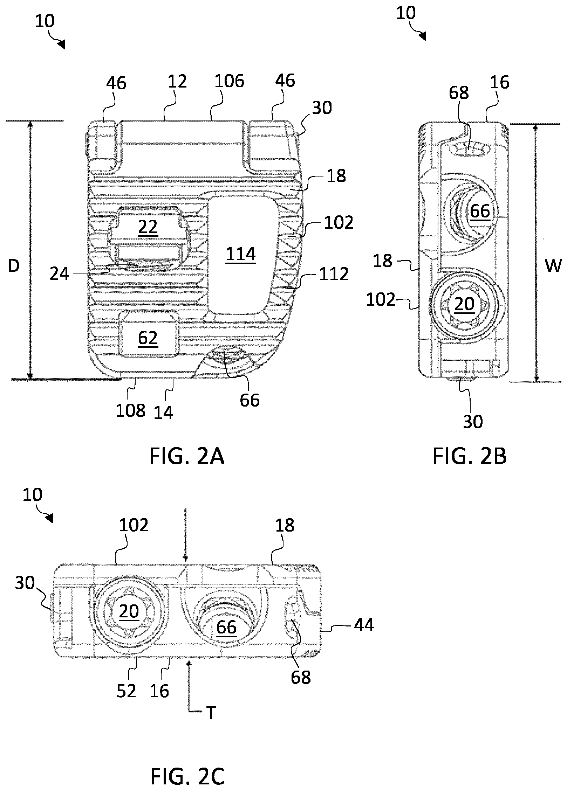

Turning now to the drawing, where like reference numerals may refer to like elements, FIGS. 1A-3 illustrate an expandable orthopedic device or implant 10, which may be configured for a Cotton procedure, according to one embodiment. The expandable implant 10 extends along a central longitudinal axis A between front end 12 and rear end 14 of the device 10. FIGS. 1A-1C show top and bottom perspective views of implant 10 in a fully expanded configuration. FIGS. 2A-2C show an adjustable thickness T and a footprint including a depth D and a width W of the implant 10 configured to correct the deformity. The wedge thickness T of implant 10 is adjustable to achieve the desired movement or correction of the bone(s). It should be understood that reference to the front and rear ends 12, 14, depth D, width W, and thickness T are described with respect to the direction of placement into a surgical site. For example, the front end 12 enters the surgical site first to a given depth D, oriented to have a given width W, for example, targeting the medial dorsal cuneiform bone in the foot for a Cotton procedure, followed by the rear end 14 of the implant 10, and expanded to a given thickness T. These and other directional terms may be used herein for descriptive purposes and do not limit the orientation(s) in which the devices may be used.

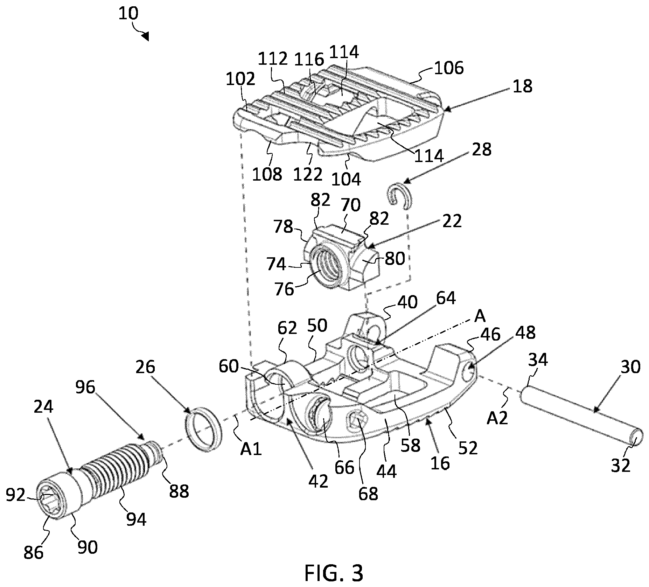

With further emphasis on the exploded view in FIG. 3, expandable implant 10 includes a main body or bottom endplate 16, a movable endplate or top endplate 18, and a drive assembly 20 configured to angulate the top endplate 18 relative to the bottom endplate 16. The drive assembly 20 may include a moveable actuator 22, a rotatable drive screw 24, a friction ring 26, and a retaining ring 28 aligned along longitudinal axis A1. In one embodiment, longitudinal axis A1 may be parallel and offset to central longitudinal axis A. The top endplate 18 may be pivotably connected to the bottom endplate 16 with a hinge pin 30 aligned along pivot axis A2, which is perpendicular to central longitudinal axis A. The hinge pin 30 may be located at the front end 12 of the implant 10 to provide the variable wedge thickness T for the implant 10 when expanded.

The bottom endplate 16 may include a front end 40 and an opposite rear wall 42. A pair of sidewalls 44 connect the front end 40 to the rear wall 42. The sidewalls 44 may be straight or curved (e.g., convexly curved). The implant 10 may form a generally quadrilateral shape, such as a rectangle or square, with curved or beveled edges. As best seen in FIG. 2A, one sidewall 44 may be straight and the opposite sidewall 44 may be outwardly curved. The front end 40 may include a pair of tabs 46 with a gap therebetween for receiving a portion of the top endplate 18. As best seen in FIG. 2A, one tab 46 may be larger or thicker than the other tab 46. The tabs 46 may define cylindrical openings 48 aligned along perpendicular axis A2, which are configured to receive the hinge pin 30 therethrough, thereby securing the top endplate 18 to the bottom endplate 16.

The bottom endplate 16 includes an inner face 50 and an opposite outer face 52. The inner face 50 may be configured to intermesh with an inner surface 104 of the top endplate 18 when collapsed, and the outer face 52 may be configured to contact bone. As best seen in FIG. 1C, a portion of the outer face 52 may include a plurality of teeth 54 or other friction increasing elements, such as ridges, roughened surfaces, keels, gripping or purchasing projections configured to retain the device 10 in bone. In one embodiment, the teeth 54 may be centrally located between flat areas or smooth surfaces 56 near the front and rear ends 40, 42. In one embodiment, the bottom endplate 16 may be 3D printed using additive manufacturing, for example, from a titanium alloy (e.g., Ti-6Al-4V or TAV). In this manner, the outer face 52 may be created with teeth and/or surface texturing that can better facilitate bony on-growth from the contacting cut surface of the bone. The 3D printed endplate 16 may allow for complex geometry to be manufactured with minimal manufacturing time.

The bottom endplate 16 may define one or more windows 58 between the inner and outer faces 50, 52. For example, several of windows 58 may be provided to align with corresponding windows 114 in the top endplate 18. One set of windows 58, 114 may align with the drive assembly 20 and another set of windows 58, 114 may be offset laterally opposite to the drive assembly 20. Any of the windows 58, 114 may be generally rectangular, oblong, circular, irregular, or of other suitable shapes to facilitate bone growth and fusion. The windows 58, 114 may be configured to receive bone graft or similar bone growth inducing material, which may be introduced within and/or around the device 10 to further promote and facilitate bone growth.

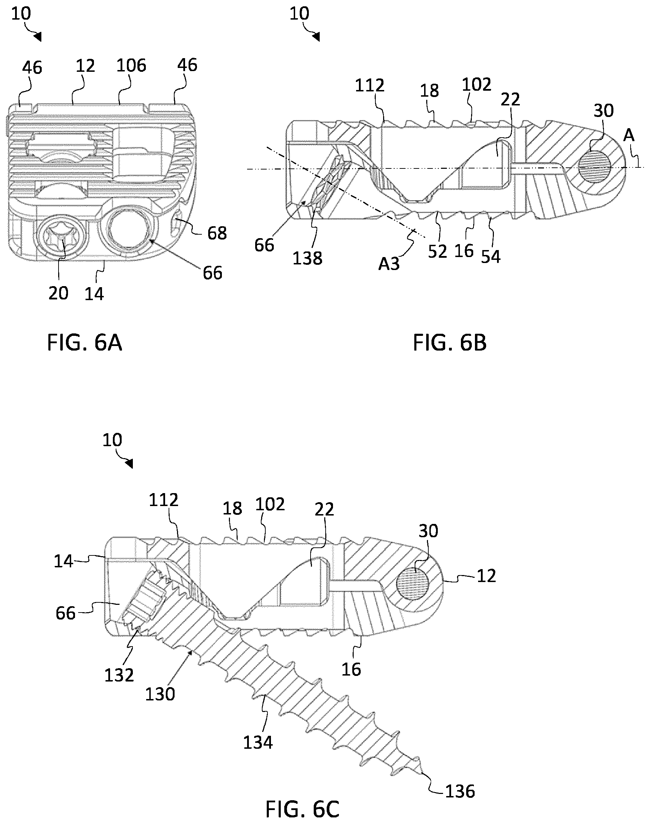

The bottom endplate 16 defines a bore 60 configured to receive a portion of the drive assembly 20. The bore 60 may be defined through the rear wall 42 and with a partial ring 62 enclosing the bore 60. A recess or pocket 64 aligned with bore 60 may be configured to receive the distal tip 88 of the drive screw 24. The bore 60 and pocket 64 may extend along offset axis A1. The bottom endplate 16 may also define a fastener opening 66 configured to receive a bone fastener 130, which secures the endplate 16 to the adjacent bone, as described in more detail with respect to FIGS. 6A-6C. The bottom endplate 16 may further define one or more instrument recesses 68 configured to be engaged by an instrument, such as an insertion instrument or implant inserter. For example, a pair of instrument recesses 68 may be provided on opposite sides of the bottom endplate 16 near the rear wall 42 to aid in implantation and/or expansion.

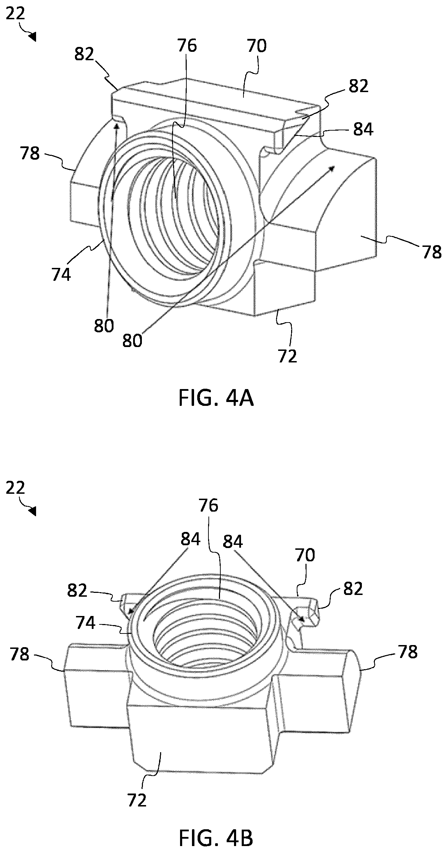

The drive assembly 20 may include an actuator 22, a drive screw 24, a friction ring 26, and a retaining ring 28, which may be aligned along longitudinal axis A1, which may be offset laterally from central longitudinal axis A. As best seen in FIGS. 4A-4B, the actuator 22 may include a block-like main body with a generally plus-shaped cross section. The actuator 22 has an upper surface 70 and an opposite lower surface 72 with a prominent cylindrical portion 74 extending from the center. The upper and lower surfaces 70, 72 may be flat or substantially planar. The cylindrical portion 74 defines a threaded through bore 76 configured for receiving the drive screw 24 therethrough. When assembled, threaded bore 76 aligns with offset axis A1. The actuator 22 includes a pair of protrusions or wings 78 on opposite sides of the cylindrical portion 74. The wings 78 may define ramps or ramped surfaces 80 on an upper portion of the wings 78, which are configured to mate with corresponding ramps 116 on the top endplate 18. The ramped surfaces 80 may be curved, rounded, or arced, thereby resembling a segment of an arc. In one embodiment, the ramped surfaces 80 may form a continuous curve from a lower position toward the rear end 14 to a higher position toward the front end 12. The arced ramps 80 on the actuator 22 may mate with angled ramps 116 on the top endplate 18 to achieve tangential surface contact at any degree of implant expansion. The actuator 22 may include a pair of tabs 82 positioned above the ramps 80 and defining a slot therebetween for receiving a portion of the top endplate 18. The tabs 82 may extend laterally outward and away from one another in the same manner as wings 78. As best seen in FIG. 4B, the tabs 82 may have an angled retainment surface 84 configured to ensure the top endplate 18 is retained at any degree of expansion. The angled retainment surface 48 may be positioned on an underside of each tab 82 with a slope or incline that rises from back to front. When approached from the rear end 14, the slope of the angled surface 84 is ascending.

The drive screw 24 extends from a proximal end 86 to a distal end 88. The proximal end 86 may include an enlarged head portion 90 configured to be received in bore 60 defined through the rear wall 42 of the bottom endplate 16. The enlarged head 90 may define an instrument recess 92 configured to receive an instrument, such as a driver, to rotate or actuate the drive screw 24. The instrument recess 92 may include a tri-lobe, hex, star, or other suitable recess configured to engage with a driver instrument to apply torque to the drive screw 24. The friction ring 26 may be seated beneath the head 90. The friction ring 26 may be a washer or annular ring, such as a polyether ether ketone (PEEK) ring, to increase friction or drag on the drive screw 24 during rotation. The friction ring 26 and drive assembly 20 may act as a lock or anti-rotation mechanism to prevent undesired implant collapse in-situ.

The drive screw 24 may include a shaft with an exterior threaded portion 94 extending along its length. The distal end 88 may have a reduced distal tip, for example, having a diameter less than the diameter of the threaded shaft 94. The distal tip 88 may define an annular groove 96 configured to receive the retaining ring 28. The retaining ring 28 may have a generally C-shaped body with an opening facing toward the bottom endplate 16. The drive screw 24 is receivable through the bore 60 in the bottom endplate 16 such that the enlarged head portion 90 of the drive screw 24 is receivable in bore 60, the threaded shaft 94 threadedly engages the threaded bore 76 through the actuator 22, and the distal tip 88 is receivable and retained in pocket 64 in the bottom endplate 16. The retaining ring 28 may be placed in the pocket 64 and dropped into the groove 96 on the screw 24 to prevent disassembly.

The top endplate 18 is pivotably connected to the bottom endplate 16 with a hinge pin 30. The hinge pin 30 is a cylindrical pin or rod extending from a first end 32 to a second end 34. When coupled to the bottom endplate 16, the first end 32 of the pin 30 is configured to be retained in the one bore 48 in tab 46 of the bottom endplate 16, the pin 30 extends through opening 110 through the top endplate 18, and the second end 34 of the pin 30 is configured to be retained in the other bore 48 in other tab 46 of the bottom endplate 16. In this manner, the top endplate 18 is configured to pivot about pivot axis A2. The hinge pin 30 may have an interference fit with one side of the bottom endplate 16 for retention, and a clearance fit with the top endplate 18 to allow the top endplate 18 to freely rotate about the hinge pin 30.

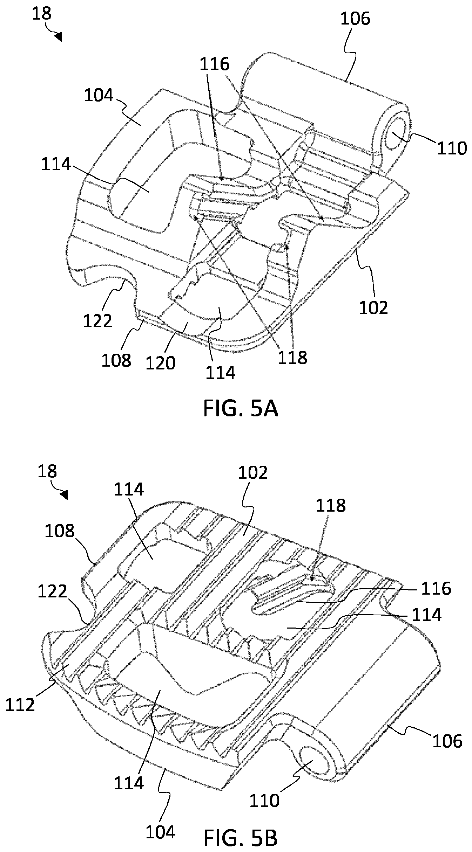

Turning now to FIGS. 5A-5B, the top endplate 18 includes an outer facing surface 102 configured to interface with bone and an opposite inner facing surface 104 configured to interface with the actuator 22 and the lower endplate 16 when collapsed. The top endplate 18 includes a front portion 106 that forms part of the implant nose and an opposite rear portion 108. The outer shape of the top endplate 18 may mimic the outer shape of the bottom endplate 16. In one embodiment, the overall implant 10 may form a generally quadrilateral shape, such as a rectangle or square, with curved or beveled edges. For example, one sidewall may be straight and the opposite sidewall may be outwardly curved to match the outer geometry of the bottom endplate 16. The front portion 106 may be rounded to facilitate insertion into the osteotomy site when collapsed and pivoting of the top endplate 18 during expansion. The front portion 106 may include an elongated cylindrical portion defining a through bore 110 configured to receive the hinge pin 30. The elongated cylindrical portion of the top endplate 18 fits between the tabs 46 of the bottom endplate 16. The through bore 110 may be aligned with perpendicular axis A2 such that the top endplate 18 is able to pivot about hinge pin 30 during expansion. In this manner, the angle between the top endplate 18 and bottom endplate 16 is able to change to a desired wedge shape and thickness.

The outer facing surface 102 of the top endplate 18 may include a plurality of teeth 112, similar to teeth 54, or other friction increasing elements, such as ridges, roughened surfaces, keels, gripping or purchasing projections configured to retain the device 10 in the bone. The teeth 112 may be provided along the entire outer facing surface 102 or a substantial portion thereof. In one embodiment, the top endplate 18 may be 3D printed using additive manufacturing, for example, from a titanium alloy (e.g., Ti-6Al-4V or TAV). In this manner, the outer face 102 may be created with teeth and/or surface texturing that can better facilitate bony on-growth from the contacting cut surface of the bone. The 3D printed endplate 18 may allow for complex geometry to be manufactured with minimal manufacturing time.

One or more openings 114 may extend through the body of the endplate 18 between the outer and inner surfaces 102, 104. In particular, a first offset opening or graft window 114 may be provided through the top endplate 18 and into fluid communication with corresponding window 58 of the bottom endplate 16. A second set of openings or windows 114 may be aligned with the drive assembly 20. The graft window(s) 58, 114 may be open and free to receive bone-graft or other suitable bone forming material. It will be appreciated that the windows 58, 114 may be of any suitable size, shape, number, and location.

The inner facing surface 104 includes one or more ramps or ramped surfaces 116 configured to interface with the corresponding ramps 80 of the actuator 22. For example, a pair of ramped surfaces 116 may be configured to slidably mate with the corresponding ramped surfaces 80 of the actuator 22. The endplate ramps 116 may be angled, diagonal, or slanted such that one end begins near inner surface 104 and extends toward rear surface 108 to terminate at a free end. The endplate ramps 80 may have a slope or incline that rises from back to front. When approached from the rear end 108, the slope of the angled ramp 116 is ascending. In one embodiment, the arced ramps 80 on the actuator 22 mate with the angled ramps 116 on the top endplate 18 to achieve tangential surface contact at any degree of implant expansion. Although the ramps are shown in a given configuration, it will be appreciated that the ramps may be sloped, slanted, or otherwise configured in any manner to provide for a desired trajectory or type of expansion. When the drive screw 24 is rotated, the actuator 22 translates toward rear end 14, thereby causing the ramped surfaces 80 of the actuator 22 to slide against the ramped surfaces 116 of the top endplate. This movement causes the top endplate 18 to pivot about pivot axis A2, lifting top endplate 18 away from bottom endplate 16, and thereby angling and increasing the wedge thickness T of implant 10.

The ramps 116 may define female channels or inner slots 118 configured to receive the mating male counterparts or tabs 82 of the actuator 22 to secure the top endplate 18 to the actuator 22, facilitating controlled movement and stable fixation at any degree of expansion. In one embodiment, the inner slots 118 may include female channels, such as a C-channel, U-channel, or J-channel. The inner slots 118 cut into the ramps 116 of the top endplate 18 slide onto the angled retainment surfaces 84 on the actuator 22 to ensure the top endplate 18 is retained at any degree of expansion. It will be appreciated that the female/male configurations may be reversed or may include other suitable ramp interactions, sliding features, or mating components to provide expansion of the top endplate 18.

The top endplate 18 may include one or more notches or cutouts 120, 122 configured to accommodate the bottom endplate 16 when collapsed and/or the trajectory of a bone fastener 130 when inserted. For example, a semi-circular cutout 120 may be defined in the inner surface 104 to accommodate a portion of the drive screw 24. A rounded notch 122 may be provided along the rear end 108 of the endplate 18 to accommodate the trajectory of the angled fastener 130 when inserted into bone.

As best seen in FIGS. 6A-6B, the bottom endplate 16 defines a fastener opening 66, which is configured to accommodate a bone fastener 130. The fastener 130 may include a head portion 132 configured to fit in the fastener opening 66 and a shaft portion 134 configured to engage bone. An outer portion of the head portion 132 may be threaded for locking engagement or non-threaded for non-locking engagement with the implant 10. The shaft portion 132 may terminate at distal tip 136, which may be sharp, blunt, or otherwise configured to engage bone. The hole geometry may be configured, for example, for fixed angle or variable angle locking screws and/or non-locking screws. In one embodiment, the fastener opening 66 defines a textured area 138, for example, having threads, ridges, bumps, dimples, serrations, knurls, or other types of textured areas, configured to lock the fastener 130 to the endplate 16. Examples of locking and non-locking holes are described in more detail in U.S. Patent No. 11,432,857, which is incorporated by reference herein in its entirety for all purposes.

In one embodiment, the fastener opening 66 is a polyaxial hole configured for placement of a locking speed screw 130 that can be inserted to prevent implant expulsion from the osteotomy site. For a Cotton procedure, the fastener opening 66 may be placed in the bottom endplate 16 and the bore axis A3 of opening 66 may be angled downward to fixate the wedge to the proximal medial cuneiform bone fragment. For an Evans procedure, a first fastener opening 66 may be placed in the bottom endplate 16 and a second fastener opening may be placed in the top endplate 18 to fixate the wedge to both calcaneus bone portions. It will be appreciated that any suitable number, type, and location of fasteners 130 may be used to secure the implant 10 in the desired surgical site.

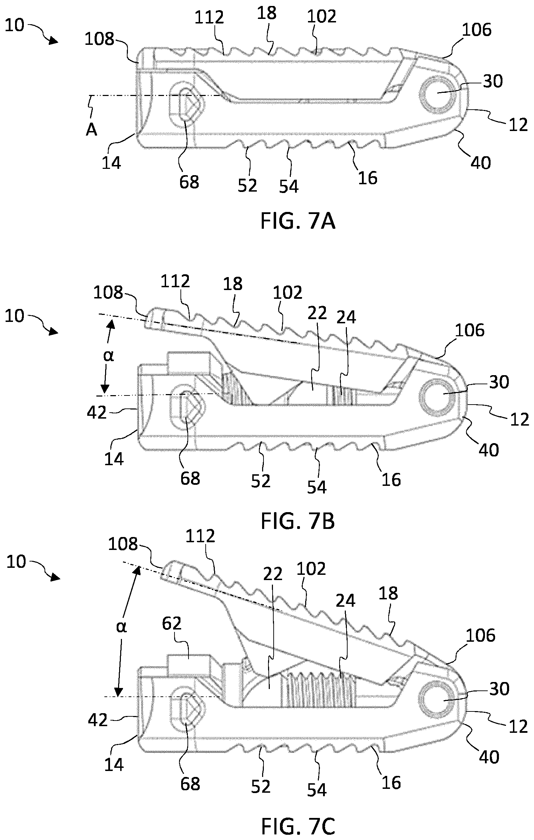

FIG. 7A shows a side view of implant 10 in a collapsed position with the top endplate 18 resting against the inner face 50 of the bottom endplate 16. In the collapsed position, there is no angle α between the top endplate 18 and the bottom endplate 16. To expand the Cotton expandable wedge 10, the drive screw 24 is rotated, which pulls the actuator 22 toward the rear 42 of the bottom endplate 16 and causes the top endplate 18 to ride up the arced ramps 80 on the actuator 22. When this happens, the angle α of the top endplate 18 relative to the bottom endplate 16 changes and increases. FIG. 7B shows a side view of implant 10 with the top endplate 18 partially expanded, for example, half-way expanded. In the partially expanded position, the rear end 108 of top endplate 18 is expanded away from the bottom endplate 16 such that a given angle α exists between the top endplate 18 and the bottom endplate 16. In this manner, the wedge thickness T of the implant 10 is increased to fill the osteotomy site and stabilize the bone. FIG. 7C shows a side view of implant 10 with the top endplate 18 fully expanded relative to the bottom endplate 16. In the fully expanded position, the rear end 108 is further expanded away from bottom endplate 16 and angle α increases, thereby providing for the greatest wedge thickness T. Due to the continuous expansions of top endplate 18, it will be appreciated that the top endplate 18 may be stopped and locked at any suitable angle α and wedge thickness T for the desired surgical outcome.

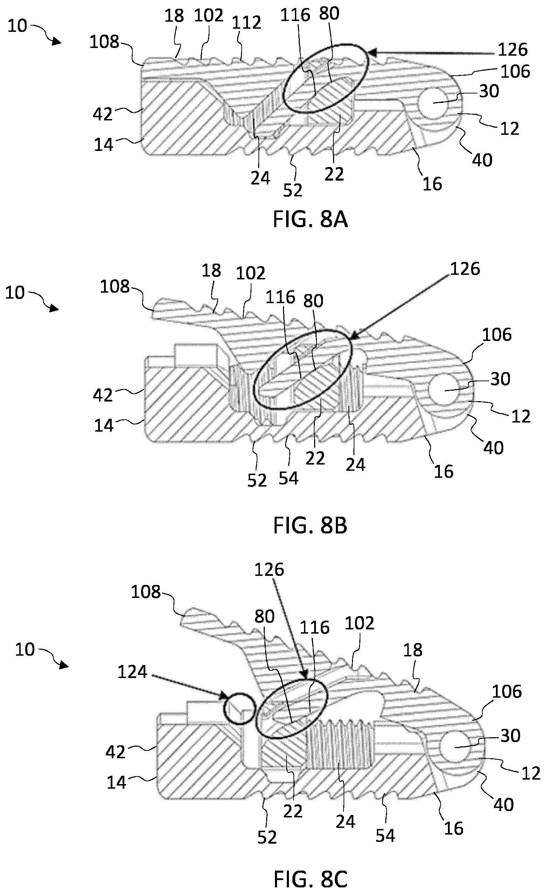

FIGS. 8A-8C show tangential sliding surface engagement 126 between the actuator 22 and the top endplate 18. In FIG. 8A, the top endplate 18 is fully collapsed against the bottom endplate 16 and the sliding surface engagement 126 is shown between ramp surfaces 80, 116. In the collapsed position, the actuator 22 is located more distally toward the front 12 of the implant 10. As shown in FIG. 8B, the top endplate 18 begins to expand away from bottom endplate 16 due to the sliding surface engagement 126. As the drive screw 24 is rotated, the actuator 22 is drawn proximally along drive screw 24 toward rear end 14. The sliding surface engagement 126 causes top endplate 18 to expand away from bottom endplate 16. FIG. 8C shows the top endplate 18 fully expanded due to the sliding surface engagement 126. At its most expanded state, the actuator 22 may hit the inner wall 124 of the cavity 58 in the bottom endplate 16 such that expansion is no longer possible. The drive screw 24 has pulled the actuator 22 toward rear wall 42, thereby fully expanding the implant 10. The sliding surface engagement 126 between the arced ramp 80 and angled ramp 116 remains tangential throughout its expansion. The tangential sliding surface engagement 126 ensures constant contact as the implant 10 adjusts to various degrees of expansion.

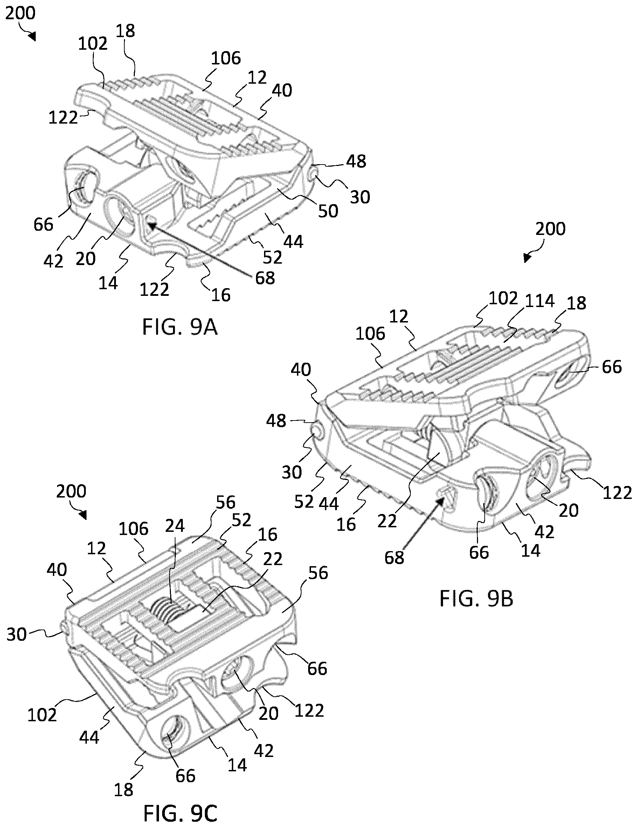

Turning now to FIGS. 9A-11, an expandable orthopedic device or implant 200, which may be configured for an Evans procedure, is shown according to one embodiment. Evans wedge 200 is similar to Cotton wedge 10 except the drive assembly 20 is centrally located and two fastener holes 66 are provided for placement of one or two bone fasteners 130 on opposite sides of the osteotomy site. Similar to the Cotton expandable wedge 10, the Evans expandable wedge assembly 200 includes bottom endplate 16, top endplate 18, actuator 22, drive screw 24, friction ring 26, retaining ring 28, and hinge pin 30.

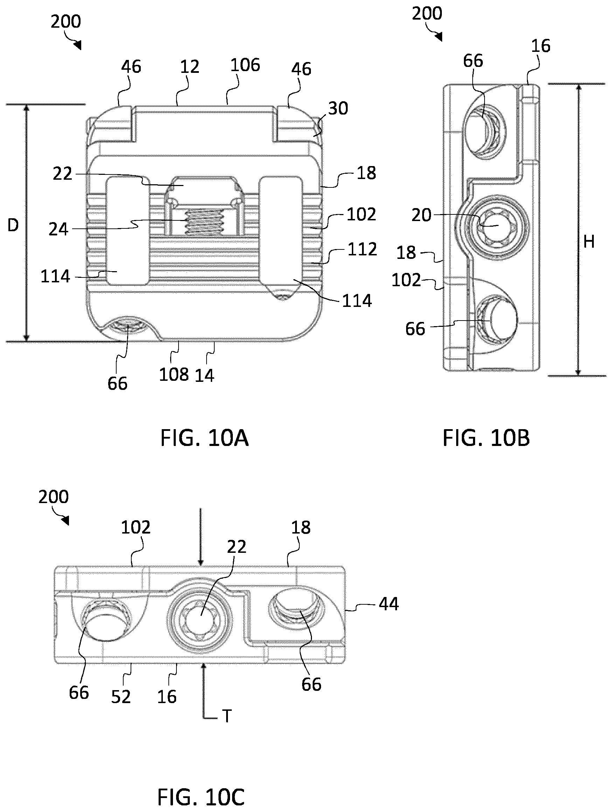

FIGS. 9A-9C show top and bottom perspective views of implant 200 in a fully expanded configuration. FIGS. 10A-10C show an adjustable thickness T and a footprint including a depth D and a height H of the implant 10 configured to correct the deformity. The wedge thickness T of implant 200 is adjustable to achieve the desired movement or correction of the bone(s). It should be understood that reference to the front and rear ends 12, 14, depth D, height H, and thickness T are described with respect to the direction of placement into a surgical site. For example, the front end 12 enters the surgical site first to a given depth D, oriented vertically to have a given height H, for example, targeting the lateral aspect of the calcaneus bone in the foot for an Evans procedure, followed by the rear end 14 of the implant 200, and expanded to a given wedge thickness T. These and other directional terms may be used herein for descriptive purposes and do not limit the orientation(s) in which the devices may be used.

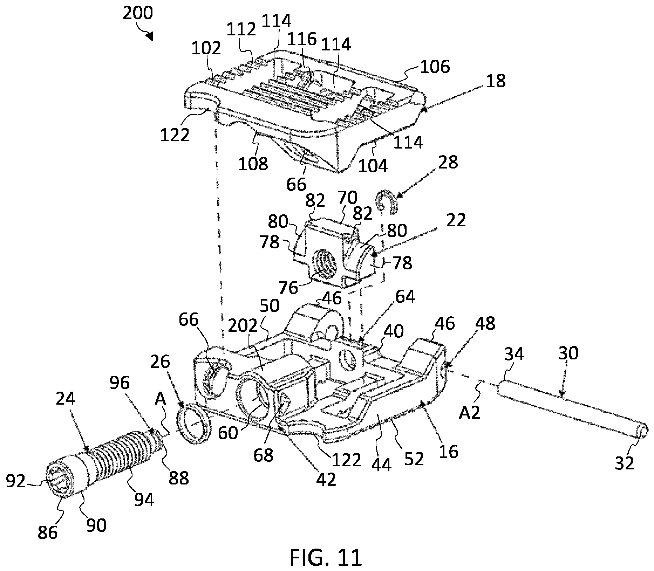

With further emphasis on the exploded view in FIG. 11, expandable implant 200 includes main body or bottom endplate 16, movable endplate or top endplate 18, and drive assembly 20 configured to angulate the top endplate 18 relative to the bottom endplate 16. In this embodiment, the drive assembly 20 including moveable actuator 22, rotatable drive screw 24, friction ring 26, and retaining ring 28 are aligned centrally along central longitudinal axis A. The bottom endplate 200 may be modified to accommodate the central drive assembly 20 and two angled fasteners 130 on opposite sides of the central drive assembly 20. For example, the partial ring 62 may be replaced with a central barrel 202 defining threaded bore 60 therethrough. The pocket 64 may be centrally located along axis A to receive the distal tip 88 of the drive screw 24. The drive screw 24 may be threaded through the actuator 22, and retained in the pocket 64 on the hinge side 12 of the bottom endplate 16 by retaining ring 28 that drops into groove 96 on the drive screw 24. Due to the modified geometry of the bottom endplate 16, one side of the barrel 202 may define the instrument recess 68 for the inserter instrument. A rounded notch 122 may be provided along the rear end 42, 108 of each endplate 16, 18 to accommodate the respective trajectories of the angled fasteners 130.

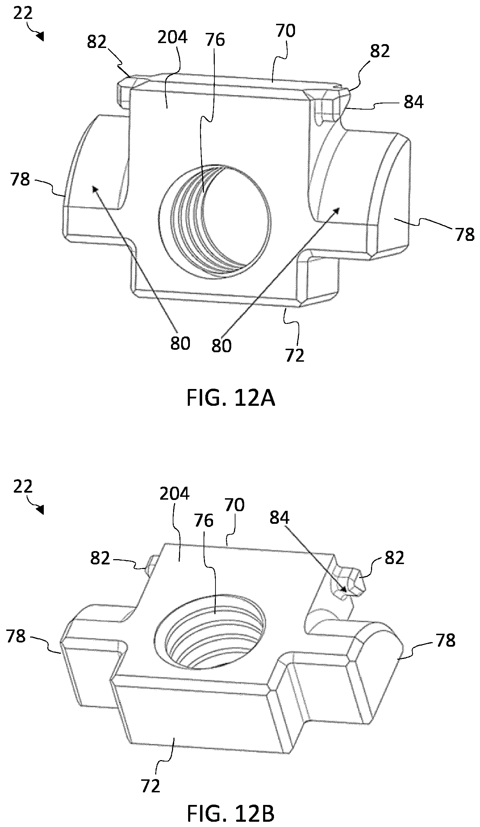

Turning now to FIGS. 12A-12B, the actuator 22 is shown in more detail. Actuator 22 has a block-like main body with a generally plus-shaped cross section. In this embodiment, the prominent cylindrical portion 74 is omitted and the rear-facing surface 204 is substantially flat or planar. The body defines threaded through bore 76 configured for receiving the drive screw 24 therethrough. When assembled, threaded bore 76 aligns with central axis A. The actuator 22 defines curved or arced ramped surfaces 80 on the wings 78, which are configured to mate with the corresponding ramps 116 on the top endplate 18. The arced ramps 80 on the actuator 22 mate with the angled ramps 116 on the top endplate 18 to achieve tangential surface contact at any degree of implant expansion. The inner slots 118 cut into the ramps 116 of the top endplate 18 slide onto the angled retainment features 84 on the actuator 22 to ensure the top endplate 18 is retained at any degree of expansion.

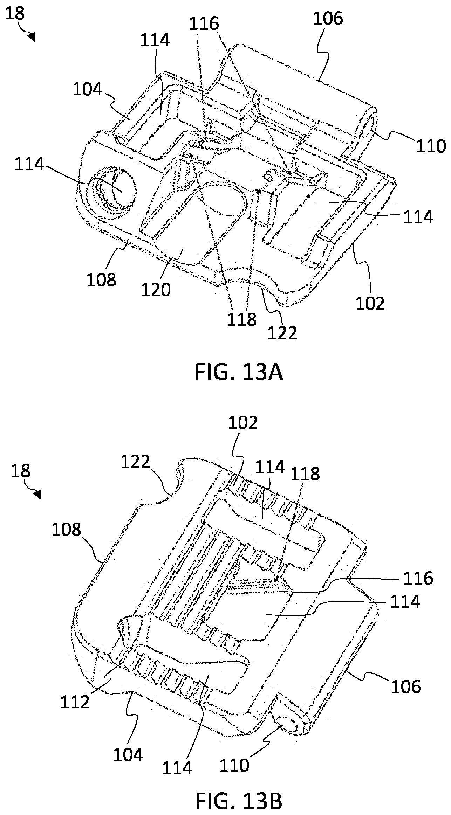

Turning now to FIGS. 13A-13B, the top endplate 18 is shown in more detail. In this embodiment, a pair of ramps 116 are centrally located about the center axis A to engage with the actuator 22. The ramps 116 may be located on opposite sides of a central through opening 114. The ramps 116 may be angled with flat outer surfaces configured to mate with the arced ramps 80 of the actuator 22. The inner slots 118 face centrally toward one another along the body of the ramps 116 and are configured to receive the angled retainment surfaces 84 on the actuator 22 to ensure the top endplate 18 is retained at any degree of expansion. The inner face 104 of the top endplate 18 may include a curved recess 120 cut out to accommodate the body of the drive screw 24. The top and bottom endplates 16, 18 may be 3D metal printed (e.g., out of TAV alloy) to achieve a surface texturing that can better facilitate bony on growth from the contacting cut surfaces of the calcaneus for the Evans procedure. The unique 3D printed design may allow for complex geometry to be manufactured with minimal manufacturing time.

The top endplate 18 may be coupled to the bottom endplate 16 by hinge pin 30, which allows the angle between the two endplates 16, 18 to change via drive assembly 20. The drive assembly 20 may incorporate an anti-rotation design to prevent undesired implant collapse. The hinge pin 30 may have an interference fit with one side of the bottom endplate 16 for retention, and a clearance fit with the top endplate 18 to allow the top endplate 18 to freely rotate about the hinge pin 30. The Evans expandable wedge 200 may be actuated from the rear side 42 to create a wedge shape, which may be engaged with an implant inserter that mates with the recesses 68 on the bottom endplate 16.

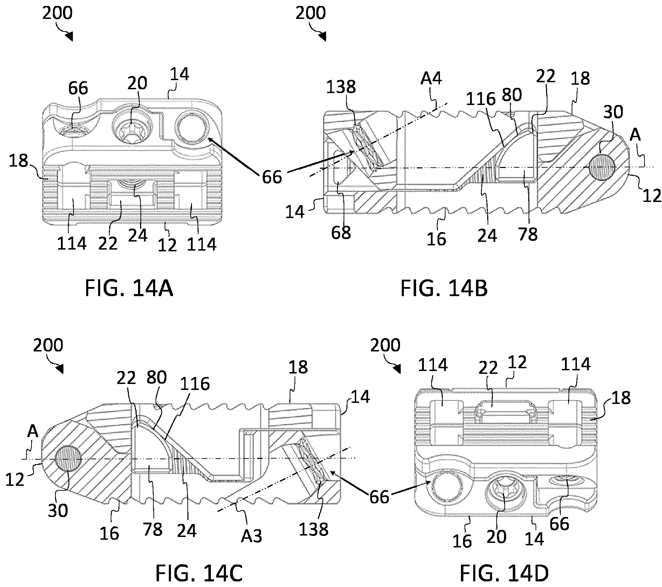

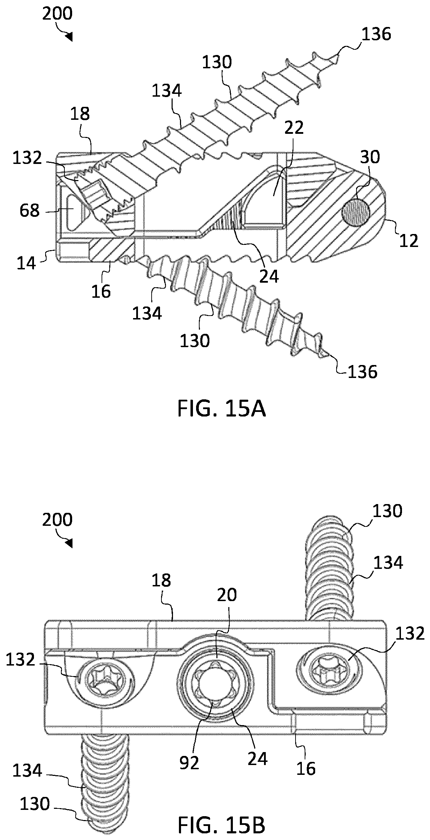

As best seen in FIGS. 14A-14D and 15A-15B, the Evans expandable wedge 200 may integrate two polyaxial holes 66 for placement of locking speed screws 130 (similar to the single one for the Cotton wedge 10). The Evans wedge 200 may have one hole 66 placed in the top endplate 18 and one hole 66 placed in the bottom endplate 16 in order to give the option to fixate the wedge 200 to the posterior and anterior calcaneal bone fragments. In one embodiment, a first bone screw 130 is angled downward along bore axis A3 into a first bone fragment and a second bone screw 130 is angled upward along bore axis A4 into a second bone fragment. The screw(s) 130 may be utilized to help prevent implant expulsion from the osteotomy site.

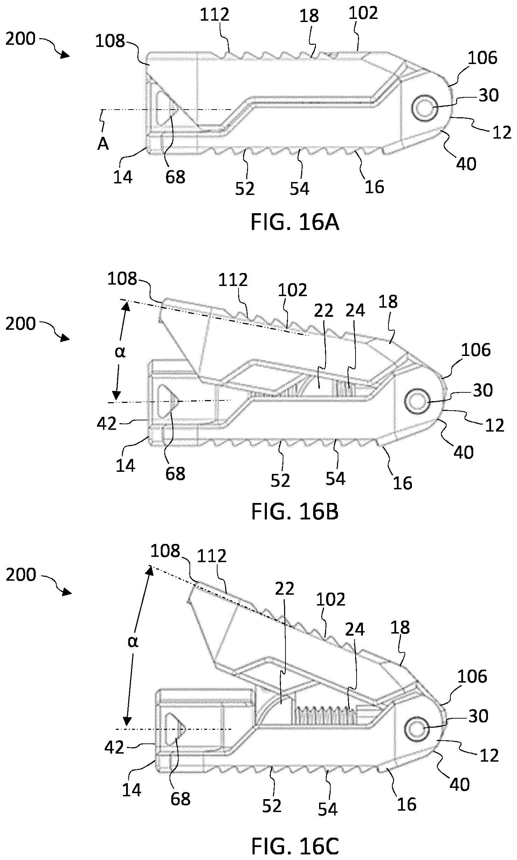

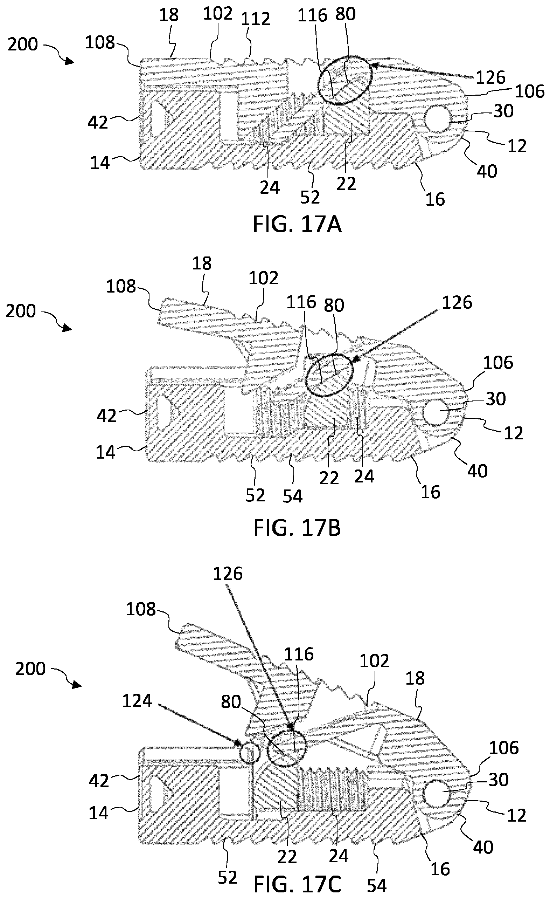

With further emphasis on FIGS. 16A-16C and 17A-17C, expansion of the implant 200 is shown in more detail. To expand the Evans expandable wedge 200, the drive screw 24 is rotated, which pulls the actuator 22 towards the back 42 of the bottom endplate 16 and causes the top endplate 18 to ride up the arced ramps 80 on the actuator 22. When this happens, the angle α of the top endplate 18 relative to the bottom endplate 16 changes and increases. At its most expanded state (shown in FIG. 17C), the actuator 22 may hit the inner wall 124 of the cavity in the bottom endplate 16 such that expansion is no longer possible.

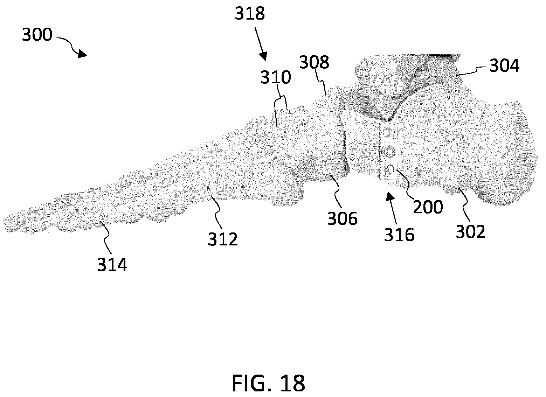

Turning now to FIG. 18, an example of a foot anatomy 300 is shown with the Evans expandable wedge implant 200 implanted into an osteotomy site 316 according to one embodiment. Anatomically, the foot 300 may be divided into bones of the hindfoot, midfoot, and forefoot. The hindfoot includes the heel bone or calcaneus 302 and the ankle bone or talus 304. The midfoot includes the cuboid bone 306, the navicular bone 308, and the cuneiforms 310. The forefoot includes the metatarsal bones 312 and phalanges 314.

During an Evans foot procedure, a cut or osteotomy 316 may be made into the calcaneus bone 302. In particular, the osteotomy 316 may be formed on the lateral aspect of the calcaneus bone 302. For example, an incision may be made on the outside of the foot 300 near the front of the ankle to access the calcaneus bone 302. The osteotomy 316 may be performed behind the calcaneal cuboid joint, thereby preserving the joint for motion. Implant 200 may be positioned in the osteotomy site 316. As shown, the implant 200 may be inserted vertically with the expandable endplate 18 facing anteriorly toward the cuboid 306. It will be appreciated that the implant 200 may also be reversed such that the expandable endplate 18 faces posteriorly toward the back of the heel. The implant 200 is expanded by actuating drive screw 24, for example, with an instrument, such as a driver. By inserting and expanding the implant 200, the lateral column of the foot 300 is lengthened, pushing the front of the foot 300 into a more neutral position. This may help to fix the valgus heel deformity by medializing the portion of the calcaneus 302 posterior to the osteotomy 316. The expandable implant 200 allows a surgeon to dial in the wedge thickness in order to achieve the desired lengthening of the lateral column of the foot 300 to correct the deformity. The implant 200 may be secured by one or both bone screws 130 to fixate the wedge to the posterior and/or anterior calcaneal bone fragments, which may help to prevent expulsion from the osteotomy site 316.

During a Cotton foot procedure, another cut or osteotomy 318 may be made into the midpoint of the dorsal aspect of the medial cuneiform 310. The osteotomy 318 may be made parallel to the first tarsometatarsal joint keeping the plantar cortex intact to create a hinge. Implant 10 may be positioned in the osteotomy site 318. The implant 10 may be inserted with the expandable endplate 18 facing anteriorly toward the metatarsals 312 or posteriorly toward the navicular 308 and talus 304. The implant 10 is expanded by actuating drive screw 24, for example, with an instrument, such as a driver. By inserting and expanding the implant 10, a plantarflexion of the forefoot is created to recreate the natural medial longitudinal arch of the foot 300. The expandable implant 10 allows the surgeon to dial in the wedge thickness in order to achieve the desired plantarflexion and correct the deformity of the foot 300. The implant 10 may be secured to the proximal medial cuneiform bone fragment with a single bone screw 130, which may help prevent expulsion from the osteotomy site 318.

Although described herein with respect to the Evans and Cotton osteotomy procedures, it will be appreciated that implants 10, 200 may also be useful for other osteotomies, such as tibial osteotomies, femoral osteotomies, pelvic osteotomies, vertebral osteotomies or resections, metatarsal/cuneiform arthrodesis, as well as other indications. It will be appreciated that the implants may also be positioned in different orientations to suit the desired surgical outcome.

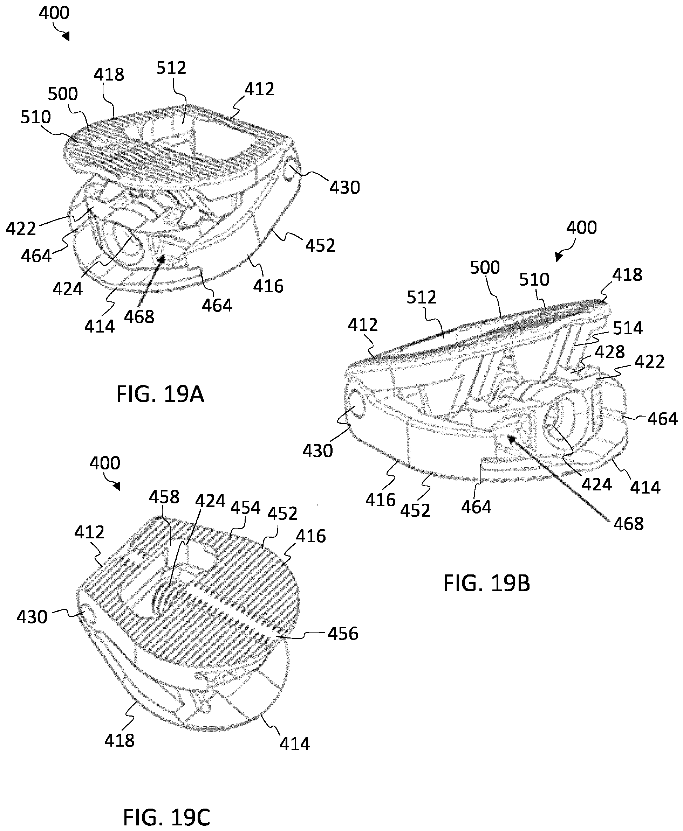

Turning now to FIGS. 19A-21, an expandable orthopedic device or implant 400, which may be configured for a Cotton procedure, is shown according to one embodiment. Implant 400 is similar to implant 10 except the functionality of the actuator 422 has been modified and two actuator pivots 428 have been added as part of the expansion mechanism. The actuator pivots 428 mate with the top endplate 418 to allow for sliding motion between the components. Each actuator pivot 428 is able to rotate to allow for continuous contact with the top endplate 418 as the angulation changes. As the actuator 422 is drawn toward the hinged side of the implant 400, the top endplate 418 travels up the actuator pivots 428 to expand the top endplate 418.

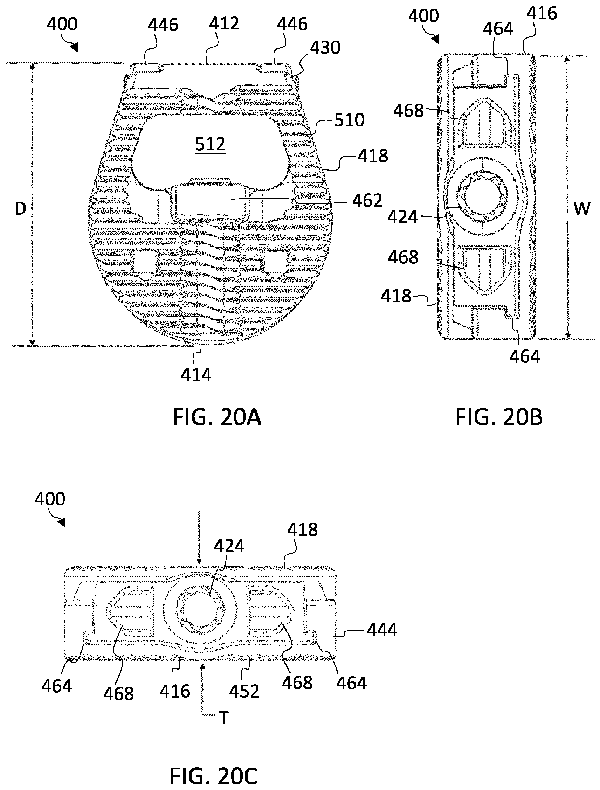

The expandable implant 400 extends along central longitudinal axis A between front end 412 and rear end 414 of the device 400. FIGS. 19A-19C show perspective views of implant 400 in a fully expanded configuration. FIGS. 20A-20C show an adjustable thickness T and a footprint including a depth D and a width W of the implant 400 configured to correct the deformity. The wedge thickness T of implant 400 is adjustable to achieve the desired movement or correction of the bone(s). It should be understood that reference to the front and rear ends 412, 414, depth D, width W, and thickness T are described with respect to the direction of placement into a surgical site, but the directional terms may be used herein for descriptive purposes and do not limit the orientation(s) in which the devices may be used.

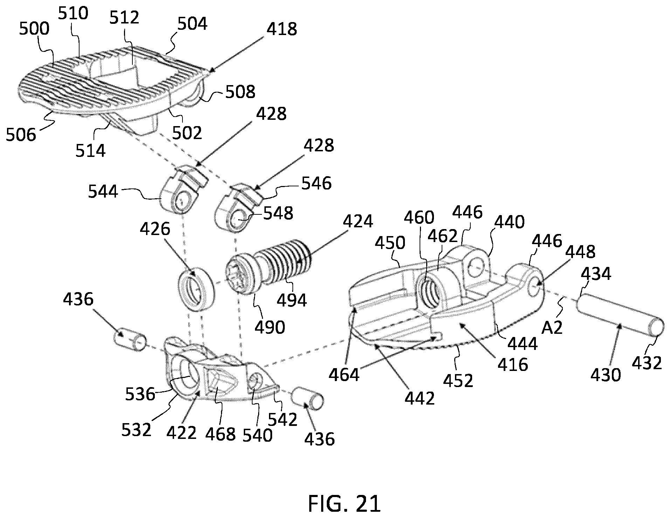

With further emphasis on the exploded view in FIG. 21, the Cotton expandable wedge assembly 400 includes a main body or bottom endplate 416, a moveable or top endplate 418, an actuator 422, two actuator pivots 428, two pivot pins 436 connecting the pivots 428 to the bottom endplate 416, a friction ring 426, a drive screw 424, and a hinge pin 430 pivotally connecting the bottom endplate 416 to the top endplate 418. The drive screw 424 and friction ring 426 may be aligned along central longitudinal axis A. The actuation mechanism may include the moveable actuator 422 and actuator pivots 428 positioned toward the rear end 414 in the closed configuration. The top endplate 418 may be pivotably connected to the bottom endplate 416 with hinge pin 430 aligned along pivot axis A2, which is perpendicular to the central axis A.

The bottom endplate 416 may include a front end 440 and an opposite rear wall 442. A pair of sidewalls 444 connect the front end 440 to the rear wall 442. The sidewalls 444 may be straight or curved (e.g., convexly curved). The implant 400 may form a generally rounded symmetrical shape or oval-shaped, such as a clam shell shape, with curved edges. As best seen in FIG. 20A, both sidewalls 444 may be outwardly curved with a taper toward the hinged side 412. The front end 440 may include a pair of tabs 446 with a gap therebetween for receiving a portion of the top endplate 418. As best seen in FIG. 20A, the tabs 446 may be equally sized and symmetrical. The tabs 446 may define cylindrical openings 448 along perpendicular axis A2, which are configured to receive the hinge pin 430 therethrough, thereby securing the top endplate 418 to the bottom endplate 416.

The bottom endplate 416 includes an inner face 450 and an opposite outer face 452. The inner face 450 may be configured to intermesh with an inner surface 502 of the top endplate 418 when collapsed, and the outer face 452 may be configured to contact bone. As best seen in FIG. 19C, a portion of the outer face 452 may include a plurality of teeth 454 or other friction increasing elements, such as ridges, roughened surfaces, keels, gripping or purchasing projections configured to retain the device 400 in bone. In one embodiment, the teeth 454 may be located along most of the outer face 452 with a central stripe 456 bifurcating two sides of the face 452. In one embodiment, the bottom endplate 416 may be 3D printed using additive manufacturing, for example, from a titanium alloy (e.g., Ti-6Al-4V or TAV). In this manner, the outer face 452 may be created with teeth and/or surface texturing that can better facilitate bony on-growth from the contacting cut surface of the bone. The 3D printed endplate 416 may allow for complex geometry to be manufactured with minimal manufacturing time.

The bottom endplate 416 may define one or more windows 458 between the inner and outer faces 450, 452. For example, a single window 458 may be centrally aligned with a corresponding window 512 on the top endplate 418 and with the drive screw 424 toward the hinged side 412 of the implant 400. Any of the windows 458, 512 may be generally rectangular, oblong, circular, irregular, or of other suitable shapes to facilitate bone growth and fusion. The windows 458, 512 may be configured to receive bone graft or similar bone growth inducing material, which may be introduced within and/or around the device 400 to further promote and facilitate bone growth.

The bottom endplate 416 defines a bore 460 configured to receive a portion of the drive screw 424. The bore 460 may be defined through a ring 462 near the center of the bottom endplate 416, which encloses the bore 460. The axis of bore 460 extends along central axis A. The bore 460 may be threaded to allow for threaded engagement with the threaded shaft 494 of the drive screw 424. The bottom endplate 416 further defines a pair of slots or grooves 464 configured to receive a portion of the actuator 422 to thereby guide translation of the actuator 422 along longitudinal axis A. The slots or grooves 464 may include long narrow channels extending from the rear face 442 of the endplate 416 in a depth toward the front end 440 along the inner sidewalls 444 of the bottom endplate 416. The grooves 464 may include narrow square-shaped openings or other suitable recesses that facilitate linear movement of the actuator 422 along the axis of the grooves 464 and prevent rotation or lateral movement. The grooves 464 may be positioned opposite to one another along the inner sidewalls 444 and aligned along a horizontal plane substantially parallel to the outer face 452 of the endplate 416. It will be appreciated that the location, number, and type of slots or grooves 464 may be modified to facilitate movement of the actuator 422.

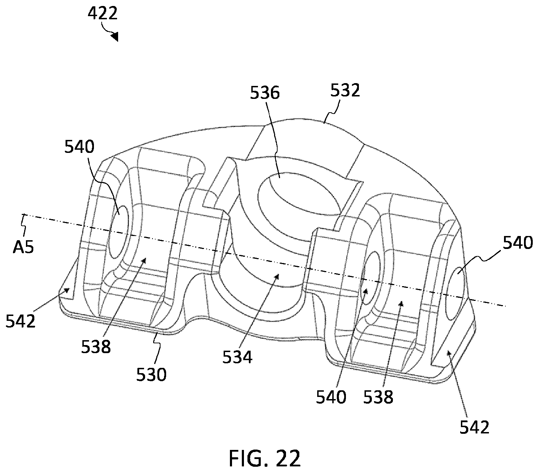

As best seen in FIG. 22, the actuator 422 includes a body having a front side 530 and a rear side 532. The actuator 422 defines a center slot 534 and a non-threaded bore 536. The center slot 534 may include a rounded concave recess extending from a top of the actuator 422 and into the body of the actuator behind the non-threaded bore 536. The center slot 534 may be sized and dimensioned to receive the assembly of the head 490 of the drive screw 424 and the friction ring 426. The axes of the center slot 534 and bore 536 are aligned with the central axis A of the implant 400. A pair of pivot slots 538 flank the center slot 534. Each pivot slot 538 may include a rounded concave recess sized and dimensioned to receive a portion of the pivot 428 therein. The sides of the actuator slots 538 may define bores or channels 540 configured to receive respective pins 436, which secure the pivots 428 to the actuator 422. The channels 540 may be cylindrically shaped to receive the corresponding cylindrical pins 436. The channels 540 may be aligned along axis A5, which may be substantially perpendicular to the central axis A and parallel to the hinge axis A2. A bottom portion of the actuator 422 includes outwardly extending tabs 542, which are receivable in the corresponding grooves 464 in the bottom endplate 416. The tabs 542 may extend laterally from the rear 530 along each side of the actuator body 422. The tabs 542 may be rectangular or square in shape projecting outward from the main body of the actuator 422 to match the corresponding inner-facing grooves 464 in the bottom endplate 416.

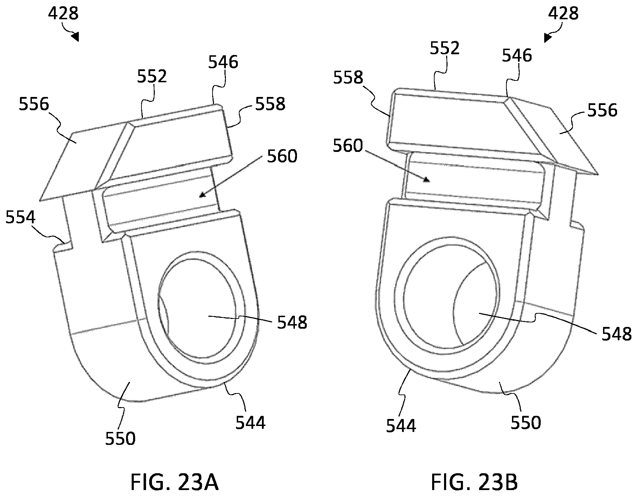

Turning now to FIGS. 23A-23B, the actuator pivots 428 are shown in more detail. Each actuator pivot 428 may include a ring 544 and a foot 546. The ring 544 may be a full annular ring defining a central through hole 548 configured to receive pivot pin 436. When assembled to the actuator 422, the axis of through holes 548 may be aligned with axis A5. Each ring 548 may have an outer surface 550 receivable in the respective slot 538 of the actuator 422. The smooth outer surface 550 allows each pivot 428 to freely rotate on the pivot pins 436 and within slot 538.

The actuator pivot 428 may include a foot 546 with a sliding surface 552 configured to mate with a corresponding sliding surface 516 on the top endplate 418. The foot 546 may be a male projection extending away from one side of ring 544. For example, the ring 544 may be truncated along one side to form a planar surface 554 to act as a base to support foot 546. The foot 546 may define an angled surface 556 on one end and a planar surface 558 on the opposite end. The foot 546 may have a slot or groove 560 around its periphery, for example, on opposite sides, thereby forming the male projection. The foot 546 is configured to enter a corresponding female ramp 514 in the top endplate 418. The parallel grooves 560 may cause portions of the foot 546 to form an overhang. The sliding surface 552 may be a smooth flat or planar surface. The sliding surfaces 552 of each pivot 428 mates with the corresponding sliding surfaces 516 on the top endplate 418 to allow for sliding motion between the components. The actuator pivots 428 are able to freely rotate on the cylindrical surface of respective pins 436, which provides continuous contact between the sliding surfaces 552 of the actuator pivots 428 and the top endplate 418 when the angulation between the top endplate 418 and bottom endplate 416 changes.

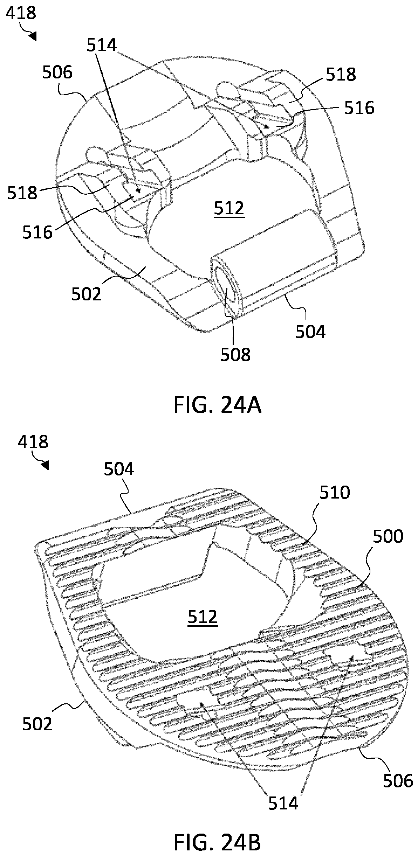

Turning now to FIGS. 24A-24B, the top endplate 418 includes an outer facing surface 500 configured to interface with bone and an opposite inner facing surface 502 configured to interface with the bottom endplate 416 and actuator pivots 428. The top endplate 418 includes a front portion 504 that forms part of the implant nose and an opposite rear portion 506. The outer shape of the top endplate 418 may mimic the outer shape of the bottom endplate 416. For example, the sidewalls may be outwardly curved with a taper toward the hinged side 412. The outer facing surface 500 of the endplate 418 may include a plurality of teeth 510 or other friction increasing elements, such as ridges, roughened surfaces, keels, gripping or purchasing projections configured to retain the device 400 in the bone. The teeth 510 may be provided along the entire outer facing surface 500 or a substantial portion thereof.

In one embodiment, the top endplate 418 may be 3D printed using additive manufacturing to achieve teeth and/or surface texturing that can better facilitate bony on-growth from the contacting cut surface of the calcaneus. The 3D printed endplate may allow for complex geometry to be manufactured with minimal manufacturing time. One or more openings 512 may extend through the body of the endplate 418 between the outer and inner surfaces 500, 502. In particular, a large central opening or graft window 512 may be provided through the top endplate 418 and into fluid communication with the graft window 458 of the bottom endplate 416. The graft window 512 may be open and free to receive bone-graft or other suitable bone forming material. The teeth 510 may be provided around the perimeter of the entire graft window 512.

The front portion 504 of the endplate 418 may include an elongate tube with a hollow channel or bore 508 configured to receive a portion of hinge pin 430. When assembled, the hollow channel 508 has a center axis aligned with pivot axis A2. When the hinge pin 430 is received through channel 508 and secured to bottom endplate 416, the top endplate 418 is able to pivot about axis A2 in order to lift the rear end 506 of the top endplate 418 relative to the bottom endplate 416. In this manner, the angle between the top endplate 418 and the bottom endplate 416 is able to change to a desired wedge shape and thickness.

The inner facing surface 502 includes one or more slides or ramps 514 configured to interface with the corresponding sliding surfaces 552 of the actuator pivots 428. For example, a pair of slides or ramps 514 may be configured to slidably mate with the slides 552 of the respective actuator pivots 428. The pair of ramps 514 may be aligned in parallel on opposite sides of the top endplate 418. The ramps 514 may be angled, diagonal, or slanted such that one end begins near inner surface 502 and extends toward front surface 504 to terminate at a free end. Although the slides or ramps are shown in a given configuration, it will be appreciated that the slides may be sloped, slanted, or otherwise configured in any manner to provide for a desired trajectory or type of expansion.

The ramps 514 may define female channels or grooves configured to receive the mating male counterparts 546 of the actuator pivots 428. In one embodiment, the ramps 514 may be a female channel, such as a C-channel, U-channel, or J-channel. Each female slide or ramp 514 may include a sliding surface 516 bounded by a pair of legs 518. The legs 518 may define an overhang or internal flange to secure the foot 546 in the channel but still allow for slidable motion with the foot 546. Each sliding surface 516 is configured to contact and allow for slidable engagement with the corresponding sliding surface 552 of the actuator pivot 428. It will be appreciated that the female/male configurations may be reversed or may include other suitable ramp interactions, sliding features, or mating components to provide expansion of the top endplate 418.

With each respective foot 546 received within each ramp 514 of top endplate 418, the sliding surfaces 552 of each actuator pivot 428 mates with the corresponding sliding surfaces 516 on the top endplate 418 to allow for sliding motion between the components. The actuator pivots 428 are able to freely rotate on the pins 436, which provide continuous contact between the sliding surface 552 of the actuator pivots 428 and the sliding surfaces 516 of the top endplate 418 when the angulation between the top endplate 418 and bottom endplate 416 changes. When expanded, the top endplate 418 pivots about pivot axis A2, lifting top endplate 418 away from bottom endplate 416, and thereby angling and increasing the wedge thickness T of implant 400.

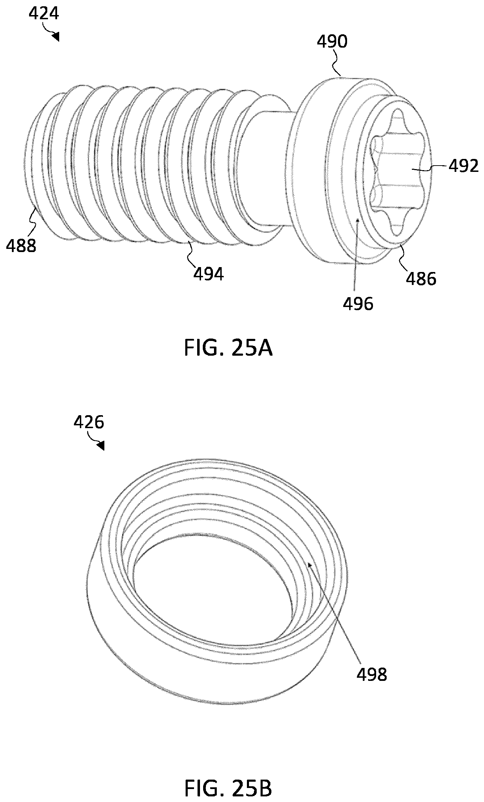

FIGS. 25A-25B show the drive screw 424 and friction ring 426 in more detail. The drive screw 424 extends from the proximal end 486 to the distal end 488. The drive screw 424 may include an enlarged head 490 defining a drive recess 492 and a shaft with an exterior threaded portion 494 extending along its length. A proximal-most portion of the drive head 490 may be recessed to form a collar 496, which is configured to seat within a corresponding pocket 498 in the friction ring 426 when assembled thereto. The pocket 498 may include an annular groove defined into the interior surface of the friction ring 426. The friction ring 426 may be assembled to the head 490 of the drive screw 424. The drive screw 424 and friction ring 426 are receivable into the center slot 536 in the actuator 422 and the shaft of the drive screw 424 is receivable through the bore 460 in the lower endplate 416 such that the threaded shaft 80 threadedly engages the threaded bore 460.

To assemble the implant 400, each pivot 428 is dropped into respective slot 538 on the actuator 422, and a pivot pin 436 is slid through each side of the actuator 422 to hold the pivots 428 in place. The pivot pins 436 may have an interference fit with one side of the actuator slot 538 for retention, and a clearance fit with the pivot 428 to allow the pivot 428 to freely rotate about the pivot pin 436. The slots 560 on either side of each pivot 428 mates with the corresponding ramped slot 514 on the top endplate 418 to allow for sliding motion between the components. Each pivot 428 is able to rotate about the cylindrical surface of the pivot pin 436, which allows for continuous contact between the sliding surfaces of the pivot 428 and the top endplate 418 when the angulation between the top endplate 418 and bottom endplate 416 changes. A portion of the drive screw head 496 seats into pocket 498 in the friction ring 426, and this assembly is dropped into the center slot 534 of the actuator 422 behind the through hole 536. The friction ring 426 may be compressed on two sides by the walls of the center actuator slot 534, and the compressed fit provides resistance to the rotating screw 424 in the form of friction. Therefore, depending on the extent of the compression, different amounts of torque may be required to overcome the static friction. The assembly including the actuator 422, pivots 428, pivot pins 436, friction ring 426, and drive screw 424 may then be slid into the bottom endplate 416 by mating the tabs 542 on either side of the actuator 422 with the grooves 464 on either side of the bottom endplate 416. The top endplate 418 is slid onto both pivots 428, and is then coupled to the bottom endplate 416 by the hinge pin 430, which allows the angle between the two endplates 416, 418 to change. The hinge pin 430 may have an interference fit with one side of the bottom endplate 416 for retention, and a clearance fit with the top endplate 418 to allow the top endplate 418 to freely rotate about the hinge pin 430.

Once fully assembled, the Cotton expandable wedge 400 may be actuated from the rear side 414 to create a wedge shape. The actuator 422 may be engaged with an implant inserter that mates with recesses 468 on the actuator 422. The actuator 422 may define one or more instrument recesses 468 configured to be engaged by an instrument, such as an insertion instrument or implant inserter. For example, a pair of instrument recesses 468 may be provided on opposite sides of the actuator 422 along the rear wall 532 to aid in implantation and/or expansion.

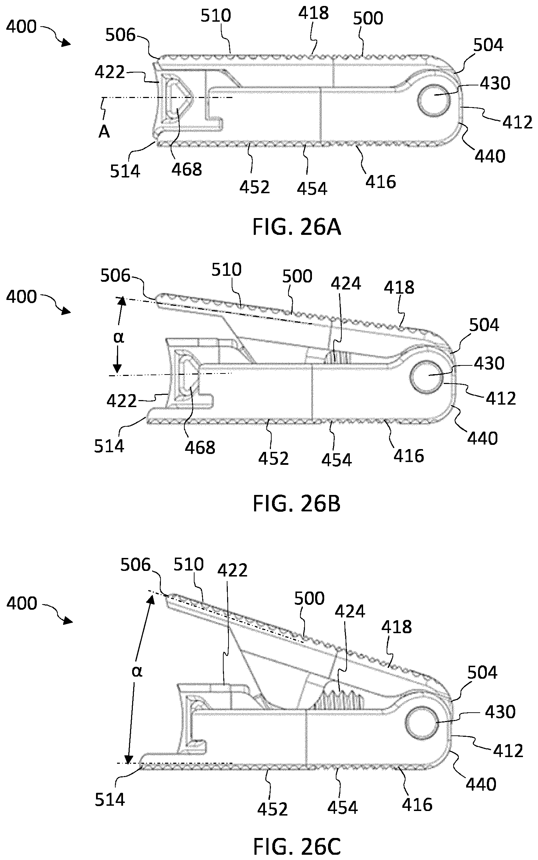

With further emphasis on FIGS. 26A-26C, expansion of the implant 400 is shown in more detail. FIG. 26A shows a side view of implant 400 in a collapsed position with the top endplate 418 resting against the inner face 450 of the bottom endplate 416. In the collapsed position, there is no angle α between the top endplate 418 and the bottom endplate 416. To expand the Cotton expandable wedge 400, the drive screw 424 is rotated, which pulls the actuator 422 toward the front 440 of the bottom endplate 416 or hinged side 412 of the implant 400 and causes the top endplate 418 to ride up the ramps 552 on the pivots 428. When this happens, the angle α of the top endplate 418 relative to the bottom endplate 416 changes and increases. FIG. 26B shows a side view of implant 400 with the top endplate 48 partially expanded, for example, half-way expanded. In the partially expanded position, the rear end 506 of top endplate 418 is expanded away from the bottom endplate 416 such that a given angle α exists between the top endplate 418 and the bottom endplate 416. In this manner, the wedge thickness T of the implant 400 is increased to fill the osteotomy site and stabilize the bone. FIG. 26C shows a side view of implant 400 with the top endplate 418 fully expanded relative to the bottom endplate 416. In the fully expanded position, the rear end 506 is further expanded away from bottom endplate 416 and angle α increases, thereby providing for the greatest wedge thickness T. Due to the continuous expansions of top endplate 418, it will be appreciated that the top endplate 418 may be stopped and locked at any suitable angle α and wedge thickness T for the desired surgical outcome.

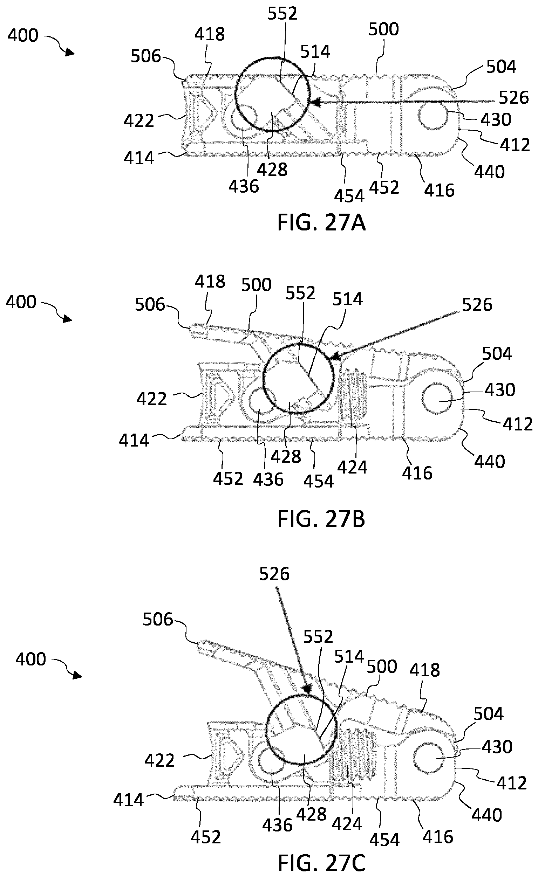

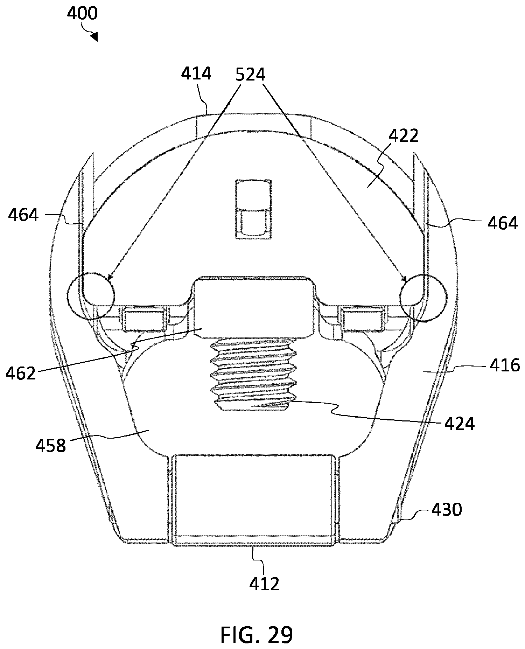

FIGS. 27A-27C show sliding surface engagement 526 between the pivots 428 and the top endplate 418. In FIG. 27A, the top endplate 418 is fully collapsed against the bottom endplate 416 and the sliding surface engagement 526 is shown between ramp surfaces 514, 552. In the collapsed position, the actuator 422 is located more proximally toward the rear 414 of the implant 400. As shown in FIG. 27B, the top endplate 418 begins to expand away from bottom endplate 416 due to the sliding surface engagement 526. As the drive screw 424 is rotated, the actuator 422 is drawn distally along drive screw 424 toward front end 412. The pivots 428 rotate and the sliding surface engagement 526 causes top endplate 418 to expand away from bottom endplate 416. FIG. 27C shows the top endplate 418 fully expanded due to the sliding surface engagement 526. At its most expanded state (as shown in FIG. 29), the actuator 422 may bottom out 524 against the curved inner walls at the back of the grooves 464 in the bottom endplate 416 such that expansion is no longer possible. The drive screw 424 has pulled the actuator 422 toward front 412, further rotating pivots 428, and fully expanding the implant 400. The sliding surface engagement 526 between the ramped surfaces 514, 552 of the pivots 428 and top endplate 418 remain in constant contact throughout expansion.

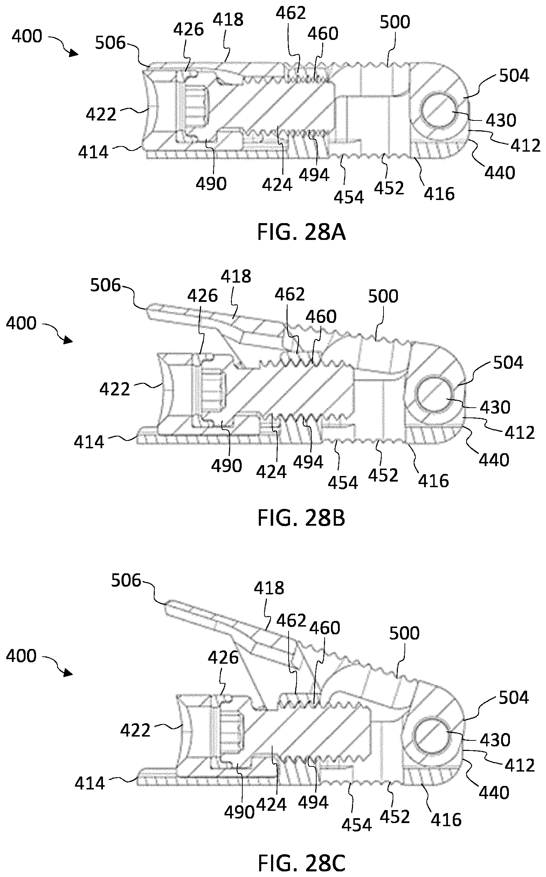

FIGS. 28A-28C show cross-sectional views of the implant 400 during expansion. In FIG. 28A, the top endplate 418 is fully collapsed and the actuator 422 is in its rear-most position. In FIG. 28B, the drive screw 424 is rotated, which pulls actuator 422 forward to rotate pivots 428 and begin to expand top endplate 418. In FIG. 28C, the drive screw 424 is further rotated, which pulls actuator 422 to its forward-most position, rotating pivots 428, and fully expanding top endplate 418 away from bottom endplate 416. Once fully expanded, the actuator 422 bottoms out 524 as shown in FIG. 29, thereby preventing any further expansion.

The expandable implants may offer a number of advantages over a static wedge. First, the expandable wedge has the ability to continuously expand over a specific range, which allows the surgeon to dial in their correction of a flatfoot deformity while performing an Evans and/or Cotton procedure. Additionally, since the expandable wedge has the capability to distract the osteotomy space while expanding, it removes the need for a secondary distracting instrument to hold open the correction at the desired final lateral column length for an Evans procedure, or at the desired degree of plantarflexion for a Cotton procedure. Second, the small starting thickness of the implant eliminates the need for trialing to determine correct implant thickness, necessary for static wedges, thereby lowering the risk of damaging walls of outer cortical shell of calcaneus and/or medial cuneiform bone that can occur when inserting/removing multiple trials. In particular, for Cotton procedures, over distraction during trialing has the potential to crack the far (plantar) cortex, causing the wedge to fit loosely in the osteotomy. The ability to insert the expandable wedge in its collapsed state lowers risk of damage to cancellous bone that surrounds the wedge when fully implanted. Once inserted, the wedge can be gently expanded to avoid bone damage. Thirdly, if revision surgery is necessary to adjust the wedge thickness to address lost correction due to bony resorption around the implant, a stab incision may be used to engage the driver with the implant rather than having to remove an entire static wedge and insert a larger one. Lastly, the flatfoot correction is a multi-faceted procedure often involving multiple bony and soft tissue correction elements. Therefore, the expandable nature of the wedges allows the surgeon to make adjustments to the Cotton and/or Evans wedge intra-operatively as each procedural element is completed.

Although the invention has been described in detail and with reference to specific embodiments, it will be apparent to one skilled in the art that various changes and modifications can be made without departing from the spirit and scope of the invention. Thus, it is intended that the invention covers the modifications and variations of this invention provided they come within the scope of the appended claims and their equivalents. It is expressly intended, for example, that all components of the various devices disclosed above may be combined or modified in any suitable configuration.

The invention can be defined inter alia by the following examples
1. An expandable implant comprising: a bottom endplate and a top endplate pivotably connected to the bottom endplate, the top endplate defining a pair of angled ramps; an actuator including a pair of wings with arced ramps configured to mate with the respective angled ramps on the top endplate; and a drive screw configured to move the actuator, wherein rotation of the drive screw translates the actuator to expand the top endplate away from the bottom endplate, wherein the arced ramps on the actuator mate with the angled ramps on the top endplate to achieve tangential surface contact at any degree of implant expansion.
2. The expandable implant of Example 1, wherein the actuator includes a prominent cylindrical portion extending from its center and defining a threaded through bore configured to receive the drive screw therethrough.
3. The expandable implant of Example 1, wherein the actuator includes a pair of tabs configured to fit within female channels defined in the angled ramps of the top endplate to retain the actuator to the top endplate.
4. The expandable implant of Example 1, wherein the top endplate is pivotably connected to the bottom endplate with a hinge pin.
5. The expandable implant of Example 1, wherein when expanded, the top endplate is angled relative to the bottom endplate.
6. The expandable implant of Example 1, wherein the bottom endplate defines a first bore configured to receive a portion of the drive screw and a second bore configured to receive a bone screw.
7. The expandable implant of Example 1, wherein the drive screw includes a threaded shaft, a distal tip with a reduced diameter configured to fit in a pocket in the bottom endplate, and an enlarged head with a drive recess.
8. An expandable implant comprising: a bottom endplate having a front end, an opposite rear end, and a pair of sidewalls connecting the front and rear ends and defining opposite sidewall grooves; a top endplate pivotably connected to the front end of the bottom endplate, the top endplate having an outer surface with a plurality of teeth configured to engage bone and an inner surface defining a pair of ramps; an actuator including a center slot and non-threaded bore, a pair of pivot slots on opposite sides of the center slot, and a pair of outwardly extending tabs receivable in the grooves in the bottom endplate to guide translation of the actuator; a pair of actuator pivots, each pivot having a ring receivable within the respective pivot slot of the actuator and secured via a pin and a foot configured to mate with one of the ramps on the top endplate; and a drive screw positioned through the non-threaded bore in the actuator and threadedly engaged with the bottom endplate, and wherein rotation of the drive screw pulls the actuator forward, thereby causing the two actuator pivots to slide along the ramps of the top endplate to expand the top endplate away from the bottom endplate.
9. The expandable implant of Example 8, wherein the foot of the actuator pivot defines a male projection with a sliding surface which mates with a corresponding sliding surface within a female portion of the ramp in the top endplate.
10. The expandable implant of Example 9, wherein the actuator pivots are able to freely rotate on a cylindrical outer surface of the respective pin, which provides continuous contact between the sliding surfaces of the actuator pivots and the top endplate when angulation between the top endplate and bottom endplate changes.
11. The expandable implant of Example 8, wherein the pivot pins have an interference fit with one side of the pivot slot and a clearance fit with the actuator pivot to allow the pivot to freely rotate about the pivot pin.
12. The expandable implant of Example 8, wherein the drive screw includes an enlarged head and a threaded shaft, and a proximal-most portion of the head is recessed to form a collar, which is configured to seat within a corresponding pocket in a friction ring.
13. The expandable implant of Example 12, wherein the head of the drive screw seats into the pocket in the friction ring and fits into the center slot of the actuator behind the non-threaded bore such that the friction ring is compressed on two sides by the center slot, which provides resistance when rotating the drive screw.
14. The expandable implant of Example 8, wherein the top endplate is pivotably connected to the bottom endplate with a hinge pin, and the hinge pin has an interference fit with one side of the bottom endplate for retention and a clearance fit with the top endplate to allow the top endplate to freely rotate about the hinge pin.
15. A method of correcting a bone deformity comprising: forming an osteotomy in bone; inserting an expandable implant into the osteotomy, the expandable implant having a bottom endplate, a top endplate pivotably connected to the bottom endplate, an actuator with curved ramps configured to engage with angled ramps on the top endplate, and a drive screw configured to move the actuator; and expanding the implant to correct the bone deformity by rotating the drive screw to translate the actuator, thereby expanding the top endplate away from the bottom endplate, wherein the curved ramps on the actuator mate with the angled ramps on the top endplate to achieve tangential surface contact at any degree of implant expansion.
16. The method of Example 15 further comprising inserting one or two bone screws through the top and/or bottom endplates to secure the implant to adjacent bone.
17. The method of Example 15, wherein the expandable implant has an adjustable wedge thickness configured to be dialed in by a surgeon to correct the deformity.
18. The method of Example 15, wherein the bone deformity is a flatfoot deformity.
19. The method of Example 15, wherein the osteotomy is formed at a midpoint of a dorsal aspect of a medial cuneiform bone during a Cotton procedure.
20. The method of Example 15, wherein the osteotomy is formed in a calcaneus bone during an Evans procedure.

## Claims

1. An expandable implant comprising:
- a bottom endplate and a top endplate pivotably connected to the bottom endplate, the top endplate defining a pair of angled ramps;
- an actuator including a pair of wings with arced ramps configured to mate with the respective angled ramps on the top endplate; and
- a drive screw configured to move the actuator, wherein rotation of the drive screw translates the actuator to expand the top endplate away from the bottom endplate, wherein the arced ramps on the actuator mate with the angled ramps on the top endplate to achieve tangential surface contact at any degree of implant expansion.

2. The expandable implant of claim 1, wherein the actuator includes a prominent cylindrical portion extending from its center and defining a threaded through bore configured to receive the drive screw therethrough.

3. The expandable implant of claim 1 or 2, wherein the actuator includes a pair of tabs configured to fit within female channels defined in the angled ramps of the top endplate to retain the actuator to the top endplate.

4. The expandable implant of any one of the preceding claims, wherein the top endplate is pivotably connected to the bottom endplate with a hinge pin.

5. The expandable implant of any one of the preceding claims, wherein when expanded, the top endplate is angled relative to the bottom endplate.

6. The expandable implant of any one of the preceding claims, wherein the bottom endplate defines a first bore configured to receive a portion of the drive screw and a second bore configured to receive a bone screw.

7. The expandable implant of any one of the preceding claims, wherein the drive screw includes a threaded shaft, a distal tip with a reduced diameter configured to fit in a pocket in the bottom endplate, and an enlarged head with a drive recess.

8. An expandable implant comprising:
- a bottom endplate having a front end, an opposite rear end, and a pair of sidewalls connecting the front and rear ends and defining opposite sidewall grooves;
- a top endplate pivotably connected to the front end of the bottom endplate, the top endplate having an outer surface with a plurality of teeth configured to engage bone and an inner surface defining a pair of ramps;
- an actuator including a center slot and non-threaded bore, a pair of pivot slots on opposite sides of the center slot, and a pair of outwardly extending tabs receivable in the grooves in the bottom endplate to guide translation of the actuator;
- a pair of actuator pivots, each pivot having a ring receivable within the respective pivot slot of the actuator and secured via a pin and a foot configured to mate with one of the ramps on the top endplate; and
- a drive screw positioned through the non-threaded bore in the actuator and threadedly engaged with the bottom endplate, and wherein rotation of the drive screw pulls the actuator forward, thereby causing the two actuator pivots to slide along the ramps of the top endplate to expand the top endplate away from the bottom endplate.

9. The expandable implant of claim 8, wherein the foot of the actuator pivot defines a male projection with a sliding surface which mates with a corresponding sliding surface within a female portion of the ramp in the top endplate.

10. The expandable implant of claim 8 or 9, wherein the actuator pivots are able to freely rotate on a cylindrical outer surface of the respective pin, which provides continuous contact between the sliding surfaces of the actuator pivots and the top endplate when angulation between the top endplate and bottom endplate changes.

11. The expandable implant of any one of claims 8 -10, wherein the pivot pins have an interference fit with one side of the pivot slot and a clearance fit with the actuator pivot to allow the pivot to freely rotate about the pivot pin.

12. The expandable implant of any one of claims 8-11, wherein the drive screw includes an enlarged head and a threaded shaft, and a proximal-most portion of the head is recessed to form a collar, which is configured to seat within a corresponding pocket in a friction ring.

13. The expandable implant of claim 12, wherein the head of the drive screw seats into the pocket in the friction ring and fits into the center slot of the actuator behind the non-threaded bore such that the friction ring is compressed on two sides by the center slot, which provides resistance when rotating the drive screw.

14. The expandable implant of any one of claims 8- 13, wherein the top endplate is pivotably connected to the bottom endplate with a hinge pin, and the hinge pin has an interference fit with one side of the bottom endplate for retention and a clearance fit with the top endplate to allow the top endplate to freely rotate about the hinge pin.
